# EUROPÄISCHE PATENTSCHRIFT

(11) **EP 0 580 689 B1**
(45) Veröffentlichungstag und Bekanntmachung des Hinweises auf die Patenterteilung: **07.02.1996**
(21) Anmeldenummer: 92908734.4
(22) Anmeldetag: 11.04.1992
(51) Int. Cl.: A61K 9/20

(54) **VERBESSERTE RETARD-SYSTEME FÜR DIE ZEITVERZÖGERTE FREIGABE MEDIZINISCHER UND/ODER BIOLOGISCHER WERTSTOFFE AUS EINEM DEPOT-TRÄGERMATERIAL**
IMPROVED RETARD SYSTEMS FOR DELAYED RELEASE OF MEDICAL AND/OR BIOLOGICAL VALUABLE SUBSTANCES FROM A DEPOT-CARRIER MATERIAL
SYSTEMES A EFFET RETARD AMELIORES POUR LIBERATION RETARDEE DE SUBSTANCES MEDICALES ET/OU BIOLOGIQUES DE VALEUR A PARTIR D'UNE MATIERE PORTEUSE DE DEPOT

(30) Priorität: 17.04.1991 DE 4112464
(43) Veröffentlichungstag der Anmeldung: 02.02.1994
(73) Patentinhaber: Henkel Kommanditgesellschaft auf Aktien, D-40191 Düsseldorf (DE)
(72) Erfinder: RITTER, Wolfgang, D-5657 Haan (DE); LEHMANN, Rudolf, D-5653 Leichlingen (DE); SORG, Rainer, D-4000 Düsseldorf 13 (DE)
(74) Vertreter: von Kreisler, Alek, Dipl.-Chem.
(86) Internationale Anmeldenummer: EP9200825
(87) Internationale Veröffentlichungsnummer: WO9218108

(56) Entgegenhaltungen:
- EP-A- 0 086 401
- EP-A- 0 290 983
- EP-A- 0 352 588
- WO-A-91/07999
- PATENT ABSTRACTS OF JAPAN vol. 13, no. 427 22. September 1989

## Beschreibung

Die Erfindung betrifft eine Weiterentwicklung auf dem Gebiet der Wirkstoffsysteme mit zeitverzögerter Freigabe von Wirkstoffen, insbesondere aus dem Bereich medizinischer und/oder biologischer Wertstoffe aus einem Trägermaterial. Kombinationsmaterialien dieser Art - und zwar sowohl aus dem Bereich pharmazeutischer Hilfsstoffe, wie biologischer Präparate, beispielsweise Pflanzenschutzmittel - sind Gegenstand zahlreicher Untersuchungen und druckschriftlicher Veröffentlichungen der jüngeren Vergangenheit. Sie werden beispielsweise unter den Begriffen Retard-Systeme, Depot- beziehungsweise Slow Release-Materialien oder ganz allgemein als Wirkstoffgemische mit verzögerter Wertstofffreigabe bezeichnet.

Depot-Materialien dieser Art bestehen in der Regel aus einem Träger mit oder ohne Eigenwirkung, in den der zeitverzögert freizusetzende Wertstoff eingearbeitet ist. Besondere Beachtung finden in der Literatur der letzten Jahre als Trägermaterial Polymerverbindungen auf Basis von Polyestern aus niederen Hydroxycarbonsäuren mit insbesondere 2 bis 6 C-Atomen im Hydroxycarbonsäuremolekül. Trägermaterialien dieser Art sind ihrerseits Hydrolyse-labil und unterliegen biologischen Abbaumechanismen. Besondere Bedeutung kann dabei entsprechenden Polyestern vergleichsweise eingeschränkten Molekulargewichts auf Basis Glykolsäure, Milchsäure und/oder Hydroxybuttersäure zukommen.

Aus dem jüngeren einschlägigen Schrifttum sei beispielsweise verwiesen auf die US-PS 4,011,312, die zur Behandlung der Rindermastitis bei Raum- und Körpertemperatur feste Zubereitungsformen von Copolyestern aus Glykolsäure und Milchsäure mit Holgewichten unter 2.000 als Trägermaterial in Abmischung mit antibiotischen Wirkstoffen wie Tetrazyklin, Neomycin und weiteren Antibiotikas offenbart. Die Herstellung von niedermolekularem Poly(beta-Hydroxybutyrat) und dessen Verwendung als festes Trägermaterial für die Formulierung von einen pharmazeutischen Wirkstoff enthaltenden Matrix-Retardtabletten wird beschrieben in "Die angewandte makromolekulare Chemie" 161 (1988) 1 - 8 (Nr. 2604). Bei der Untersuchung der Liberationskinetik wird im hier dargestellten Fall gezeigt, daß die freigesetzte Wirkstoffmenge von der Molmasse der verwendeten PHB-Fraktion stark abhängig ist, wobei die Retardierung in der Freisetzung des Wirkstoffes mit abnehmendem Molgewicht zunimmt.

Zahlreiche Untersuchungen beschäftigen sich mit der Verwendung von resorbierbaren Polyestern auf Basis Glykolsäure/Milchsäure als Trägermaterial mit verzögerter Wirkstofffreisetzung bei ihrem Einsatz gemeinsam mit medizinischen Wertstoffen, Pflanzenschutzmitteln und dergleichen. Verwiesen wird beispielsweise auf D.L. Wiese et al. in: Drug Carriers in Medizine, Academic Press London 1979, S. 237 - 270 und die dort zitierte Literatur zu Retardformen von Arzneimitteln; R.L. Kronenthal, Polym. Sci. Technol. 1975, 8 (Polym. Med. Surg.) 119 - 137.

Die Art und Weise der Wirkstofffreigabe wird einerseits durch die Interaktion zwischen Wirkstoff und Trägermaterial bedingt, hängt aber andererseits damit stark von der Struktur und den Eigenschaften des üblicherweise polymeren Trägermaterials ab. Der Wirkstoff kann beispielsweise in Polyestern der genannten Art mikroverkapselt sein, er kann in die Polymermatrix eingebettet sein oder an die Endgruppen gebunden werden.

Die Freigabe des Wirkstoffes erfolgt damit je nach der vorgegebenen Formulierung aufgrund verschiedener Mechanismen. Bei verkapselten Wirkstoffen spielt die Diffusion der Wirkstoffe durch Mikroporen in der Umhüllung eine wichtige Rolle. Bei Dispersion oder Lösung der Wirkstoffe in einer festen Polymermatrix wird die Freigabe der Wirkstoffe über die Diffusion durch die Matrix oder über die hydrolytische Erosion der Matrix oder eine Kombination dieser Parameter bestimmt. In die Betrachtungen zur Brauchbarkeit eines solchen Depot-Systems gehen aber auch Überlegungen zur Handhabbarkeit des jeweils betroffenen Stoffgemisches ein. Das Depot-Material soll unter normalen Lagerungsbedingungen lagerbeständig sein, hinreichend fest und damit portionierbar vorliegen, unter den praktischen Bedingungen der Herstellung, des Transports und der Lagerung nicht verklumpen, hinreichende Beständigkeit gegen den unvermeidlichen Feuchtigkeitszutritt aufweisen und dergleichen.

Unter Berücksichtigung dieser zahlreichen, nur schwer miteinander zu vereinbarenden Anforderungen ist es nicht verwunderlich, daß bis heute im praktischen Einsatz Retard-Systeme beziehungsweise Depot-Materialien auf Basis von körperresorbierbaren Polyestern niederer Hydroxycarbonsäuren nicht bekannt sind.

Die Erfindung geht von der Aufgabe aus, Retard-Systeme beziehungsweise Depot-Materialien der geschilderten Art dergestalt weiter zu entwickeln, daß eine optimale Anpassung des einzusetzenden Fertigproduktes für den jeweiligen Anwendungsfall möglich wird. Die Erfindung stellt im Sinne eines Baukastensystems die im nachfolgenden im einzelnen geschilderten Elemente zum Aufbau des Depot-Materials zur Verfügung, die dann in Anpassung an das jeweils bestehende Anforderungsprofil einen bisher nicht gekannten Freiheitsgrad für den Aufbau des jeweils konkret geforderten Eigenschaftsbildes im Fertigprodukt ermöglichen.

### Gegenstand der Erfindung

Gegenstand der Erfindung ist in einer ersten Ausführungsform die Mitverwendung von radikalisch reaktiven Komponenten auf Basis olefinisch ungesättigter Ester niederer Hydroxycarbonsäuren mit 2 - 6 C-Atomen und/oder deren Oligomeren zur Modifizierung von Depot-Materialien, insbesondere Retard-Systemen, die medizinische und/oder biologische Wertstoffe in Abmischung mit einem Trägermaterial bei zeitverzögerter Wirkstoffabgabe im praktischen Einsatz enthalten. Diese Reaktivkomponenten liegen dabei in der Applikationsform des Retard-Systems als Polymerverbindungen vor und können dabei in weitgehend homogener oder auch ungleichmäßiger Verteilung im zu applizierenden Retard-System beziehungsweise Depot-Material angeordnet sein.

Gegenstand der Erfindung sind dementsprechend insbesondere weiterhin medizinische und/oder biologische Wertstoffe enthaltende Retard-Systeme auf Basis von Oligomer- und/oder Polymerverbindungen niederer Hydroxycarbonsäuren mit 2 bis 6 C-Atomen als Trägermaterial mit verzögerter Wirkstofffreigabe, wobei das Kennzeichen der Erfindung darin liegt, daß diese Retard-Systeme beziehungsweise Depot-Materialien mit Polymerverbindungen hergestellt aus olefinisch ungesättigten Estern niederer Hydroxycarbonsäuren mit 2 bis 6 C-Atomen und/oder deren Oligomeren modifiziert sind. Diese im Sinne der erfindungsgemäßen Lehre Polymerverbindungen bildenden olefinisch ungesättigten Ester niederer Hydroxycarbonsäuren und/oder deren Oligomeren werden im Rahmen der Erfindungsbeschreibung der Einfachheit halber auch als "Vernetzer" oder ''Vernetzerkomponenten" bezeichnet.

### Einzelheiten zur Erfindung

Das System der erfindungsgemäßen Lehre wird im nachfolgenden an Träger/Wertstoff-Gemischen beschrieben, die für den Einsatz am Menschen und/oder Tier bestimmt sind und antibiotisch wirksame Wertstoffe - insbesondere sogenannte Breitbandantibiotika - in einem körperresorbieren Trägermaterial auf Basis niederer Hydroxycarbonsäuren - insbesondere auf Milchsäure- und/oder Glykolsäurebasis - enthalten. Das im nachfolgenden beispielhaft im einzelnen geschilderte Kombinationsmaterial ist dabei dergestalt gewebeverträglich ausgebildet, daß seine Inkorporation in den lebenden Körper möglich ist. Besondere Bedeutung besitzt dieses Material als Retard-System für die verzögerte und hinreichend lang anhaltende Antibiotikafreigabe bei der Implantation des erfindungsgemäß beschriebenen Mehrkomponentenmaterials in lebendes menschliches oder tierisches Gewebe. Dieses Retard-Material im Sinne der Erfindung ist damit als körperresorbierbarer Hilfsstoff zur Wunddesinfektion bei operativen Eingriffen geeignet. Ersetzt werden können damit die bis heute üblichen nicht resorbierbaren Hilfsmittel, die beispielsweise eine verzögerte Antibiotikafreigabe aus in den Operationsbereich eingelagerten Feststoffkörpern sicherstellen, dann aber aus dem menschlichen oder tierischen Körper wieder entfernt werden müssen.

Diese Darstellung der Erfindung an dem hier geschilderten konkreten Beispiel beabsichtigt keine Einschränkung der erfindungsgemäßen Lehre in diese bestimmte Richtung. Es werden vielmehr an diesem Beispiel nur die Kombinationselemente des erfindungsgemäßen Handelns erläutert, die sinngemäß für andere Anwendungsgebiete analog anwendbar sind. So kann insbesondere im Rahmen der erfindungsgemäßen Lehre eine beliebige Wahl der freizusetzenden Wertstoffe vorgegeben sein. Wichtige weitere pharmakologische Wertstoffe für die Implantation in den lebenden Körper sind beispielsweise Zytostatika, Hormone, Insulin, Cortison und dergleichen. Durch Einbettung des jeweiligen Depot-Materials in den von der Erkrankung betroffenen oder einen anderen frei gewählten Bereich können einstellbar hohe lokale Wirkstoffkonzentrationen über einen längeren Zeitraum bei gleichzeitiger Absenkung der Gesamtbelastung des Organismus erreicht werden.

Der Anwendungsbereich der erfindungsgemäßen Retard-Systeme im medizinischen beziehungsweise veterinär-medizinischen Bereich ist aber nicht auf Wertstoff-enthaltende Stoffmischungen für die unmittelbare Implantation in lebendes Gewebe eingeschränkt. Die Erfindung umfaßt gleichermaßen Wertstoff-enthaltende Abmischungen, die zur beliebigen topischen Verabreichung am Mensch oder Tier geeignet sind. Der Begriff der topischen Verabreichung umfaßt dabei sowohl die Wertstoffapplikation über Schleimhautbereiche als auch die sogenannte transdermale Wertstoffverabreichung durch Aufbringen der entsprechenden Präparate auf ausgewählte Bereiche der Außenhaut. In allen diesen Fällen zeichnen sich Stoffgemische der erfindungsgemäßen Art durch die Möglichkeit der einstellbar verzögerten Wertstofffreigabe aus. Es gelingt damit beispielsweise die Wertstoffapplikation in den lebenden Organismus über einen vorbestimmbaren Zeitraum bei ebenfalls weitgehend frei vorbestimmbaren Abgabekonzentrationen. Begreiflicherweise bedarf es in jedem Einzelfall der Untersuchung und Bestimmung der Interaktionen zwischen den Bauelementen des Retard-Systems im Sinne der erfindungsgemäßen Lehre und den daraus freizusetzenden Wertstoffen sowie der Interaktion zwischen dem lebendem Körper und dem Retard-System am Ort seiner Applikation. Die große Freizügigkeit in der Auswahl der Bauelemente für die Zusammenstellung der erfindungsgemäß definierten Retard-Systeme im Zusammenwirken mit der erfindungsgemäß bestehenden vergleichsweise großen Freiheit in der Kombination dieser Bauelemente schafft aber neue Möglichkeiten für die zeitgesteuerte Freigabe und Applikation praktisch beliebiger Wertstoffe.

Die Lehre der Erfindung eignet sich insbesondere auch für Verbesserungen auf dem Gebiet der sogenannten bioadhäsiven Wirkstoffzubereitungen. Hierbei handelt es sich bekanntlich um Systeme die durch Interaktion funktioneller Gruppen beziehungsweise funktioneller Bereiche an der Oberfläche des verabreichten Wirkstoffgemisches mit Schleimhautbereichen des Körpers unter Mitwirkung von Körperflüssigkeit eine erhöhte Haftfestigkeit zeigen und damit beispielsweise eine verlängerte Verweildauer im Gastrointestinaltrakt besitzen. Zum Begriff dieser bioadhäsiven Stoffzubereitungen wird beispielsweise verwiesen auf Herve Tournier et al. "New Bioadhesive Polymers for Topical Mucosal Dosage Forms" Proceed. Intern. Symp. Control. Rel. Bioact. Mater. 15 (1988), Controlled Release Society, Inc., No. 237, S. 418/419 sowie die dort zitierte Literatur.

Je nach der konkreten Ausgestaltung des Retard-Systems und seiner Abstimmung auf die parenterale, enterale oder sonstige topische Applikation wird durch die Lehre der Erfindung eine Vielzahl von konkreten Stoffgemischen umfaßt. Alle diese unterschiedlichen Stoffgemische werden aber durch die einheitlichen Prinzipien der Erfindung beherrscht, die im nachfolgenden im einzelnen dargestellt sind.

### Das Trägermaterial für die verzögerte Wirkstofffreigabe

Die bevorzugten Trägermaterialien im Sinne der erfindungsgemäßen Lehre sind als Oligomer- und/oder Polymerverbindungen von Hydroxycarbonsäuren mit 2 bis 6 C-Atomen zu definieren, wobei entsprechenden Polyestern der Hydroxycarbonsäuren mit 2 bis 4 C-Atomen besondere Bedeutung zukommt. Entsprechende Verbindungen der Glykolsäure und/oder der Milchsäure sind nicht nur großtechnisch leicht zugängliche Verbindungen der hier betroffenen Art, aufgrund ihrer unterschiedlichen Stabilität gegen hydrolytischen Einfluß spielen Auswahl Und Abstimmung dieser Komponenten aufeinander eine wichtige Rolle. Der Begriff der Milchsäurederivate umfaßt dabei die entsprechenden Verbindungen beliebiger sterischer Konfiguration, also die D-, L- und/oder DL-Formen einschließlich der Racemate. In an sich bekannter Weise wird durch die jeweilige Wahl des beziehungsweise der polyesterbildenden Molekülbaustein(e) die Abbauzeit der Polymerverbindung unter dem hydrolytischen Einfluß des Körpergeschehens bestimmt. So wird bekanntlich Polyglykolsäure innerhalb weniger Tage durch hydrolytischen Abbau zersetzt, während die Abbauzeit von Poly-DL-Lactid im Bereich von Wochen bis einige Monate liegen kann.

In einschlägigen Veröffentlichungen der jüngeren Vergangenheit wird dementsprechend entscheidend Wert gelegt auf die Einstellung bestimmter und gegebenenfalls möglichst einheitlicher Polymerisationsgrade in Polymermaterialien der hier betroffenen Art, verwiesen sei beispielsweise auf die EP-A1 0 244 114 und die EP-A2 0 299 730. Hier wird versucht durch Auswahl und Einstellung ausgewählter Parameter des Trägermaterials auf Polyesterbasis das vielgestaltige Anforderungsprofil an das Depot-Material zu erfüllen.

Die Lehre der Erfindung ermöglicht über die Mitverwendung der zuvor definierten und im Nachfolgenden noch im einzelnen geschilderten Vernetzerkomponenten als integraler Bestandteil des Depot-Materials die weitgehend freie Variation des hier betroffenen Trägers auf Basis der niederen Hydroxycarbonsäuren mit 2 - 6 C-Atomen, insbesondere der Milchsäure und/oder Glykolsäure. Die Lehre der Erfindung ermöglicht hier insbesondere bei der Auswahl der Hauptmasse der Trägersubstanz deren Festlegung im Sinne einer Optimierung der verzögerten Wertstofffreisetzung unter den jeweiligen Anwendungsbedingungen. Weitere Elemente des insgesamt geforderten Anforderungsprofils an das Depot-Material werden durch die mitverwendeten Vernetzerkomponenten in der noch zu schildernden Weise geschaffen beziehungsweise erfüllt.

Geeignete Trägermaterialien für die Lehre der Erfindung sind damit allgemein Oligomere und/oder Polymere der niederen Hydroxycarbonsäuren mit 2 - 6 C-Atomen mit zäh-viskoser bis fester Beschaffenheit bei Raumtemperatur. Geeignet können insbesondere Oligoester mit mittleren Molekulargewichten im Bereich von etwa 200 bis 5.000 sein, wobei bevorzugte Oligoestermaterialien mittlere Molekulargewichte im Bereich von etwa 300 bis 2.000 besitzen.

In den der Erfindung zugrundeliegenden Arbeiten hat sich überraschenderweise gezeigt, daß besonders praxisgerechte Wirkstofffreigaben beim Einsatz in Kombination mit Antibiotika dann eingestellt werden können, wenn die Hauptmasse der Trägersubstanz auf Basis sogenannter Knochenwachse aufgebaut ist, wie sie in den Druckschriften DE 32 29 540, DE 37 16 302 und DE 38 25 211 beschrieben sind. Die Offenbarung dieser Druckschriften zur Beschaffenheit der dort beschriebenen zäh-viskosen bis festen wachsartigen Polyester-Oligomeren wird hiermit ausdrücklich auch zum Gegenstand der vorliegenden Erfindungsoffenbarung im Zusammenhang mit der Ausgestaltung des Trägermaterials mit verzögerter Wirkstofffreigabe gemacht.

Die DE 32 29 540 beschreibt resorbierbare Wachse zur mechanischen Blutstillung an körpereigenem Hartgewebe aus den genannten niederen Hydroxycarbonsäuren, insbesondere der Glykolsäure und/oder der Milchsäure. Aufgrund ihrer Struktur sind diese Wachse durch körpereigene Stoffwechselmechanismen abbaubar, wobei die Geschwindigkeit des Abbaus in an sich bekannter Weise einstellbar ist. Oligomere der Glykolsäure werden vom körpereigenen Stoffwechsel schneller abgebaut als solche der Milchsäure. Die bevorzugten Wachse weisen mittlere Molekulargewichte im Bereich von etwa 200 bis 1.500 und insbesondere im Bereich von etwa 300 bis 1.000 auf.

Zur Regelung des mittleren Molekulargewichts dieser Polyester-Oligomeren wird vorgeschlagen, monofunktionelle und/oder mehrfunktionelle Alkohole oder Carbonsäuren mitzuverwenden. In an sich bekannter Weise kann dann durch Wahl geeigneter Mischungsverhältnisse von Oxycarbonsäure und zusätzlicher nonofunktioneller beziehungsweise mehrfunktioneller Komponente ein mittleres Molekulargewicht vorherbestimmt werden. Die anfallenden Reaktionsprodukte enthalten noch gewisse Anteile an eingesetzten Ausgangskomponenten.

Die DE 37 16 307 beschreibt die weiterführende Optimierung dieser wachsartigen Polyester. Beschrieben ist hier insbesondere, daß zur Einstellung des mittleren Molekulargewichts ein ganz bestimmter dreifunktioneller Alkohol, nämlich Glycerin, eingesetzt wird. Die Kombination von Glycerin mit Oligoestern der Milchsäure und/oder der Glykolsäure führt zu abbaubaren wachsartigen Komponenten der genannten Art, die sich bei der Implantation in lebendes Körpergewebe durch eine besonders gut ausgeprägte Körperverträglichkeit auszeichnen. Zum Ausschluß unerwünschter Gewebsschädigungen kann eine Reinigung des abbaubaren Wachses unter Entfernung nicht reagierter Carboxylgruppen vorgesehen sein.

Die körperresorbierbaren zäh-viskosen bis festen Wachse der DE 38 25 211 auf Basis von Oligomeren der Glykolsäure und/oder der Milchsäure zeichnen sich schließlich durch einen Gehalt an körperverträglichen Salzen organischer und/oder anorganischer Säuren aus, die durch entsprechende Abreaktion der im Oligomerwachs gegebenenfalls vorliegenden freien Carboxylgruppen ausgebildet und/oder als zugesetzte Salze homogen verteilt in das Wachs eingearbeitet sind. Geeignet sind als Mischungskomponenten insbesondere entsprechende Alkali-, Erdalkali- und/oder Aluminiumsalze, wobei besondere Bedeutung den Salzen des Natriums, Calciums und/oder Magnesiums zukommt.

In einer bevorzugten Ausführungsform der Erfindung werden als Trägermaterialien oligomere Wachse des Bereichs vergleichsweise niedriger Molekulargewichte eingesetzt. Hachse dieser Art basierend ausschließlich auf Glykolsäure als niedere Hydroxycarbonsäure zersetzen sich jedoch so schnell, daß nur ein vergleichsweise kurzer Zeitraum der verzögerten Wertstofffreigabe sichergestellt werden kann. Im Sinne des erfindungsgemäßen Baukastenprinzips kann in einem Teilaspekt davon sinnvoll Gebrauch gemacht werden: Der Einbau von solchen Glykolsäure-basierten Wachsanteilen in das Retard-System führt in der Anfangsphase des Wirkungszeitraums zu Wertstofffreisetzungen kontrollierbarer Höhe. So kann es in der operativen Technik wünschenswert sein, in den ersten ein oder zwei Tagen nach dem operativen Eingriff lokal eine vergleichsweise hohe Antibiotikakonzentration sicherzustellen. Die Zugabe entsprechender Anteile eines sich rasch zersetzenden Glykolsäurewachses, das mit den vorgegebenen Antibiotikamengen gefüllt ist, stellt dieses Anforderungsprofil sicher.

Für die längerwirkende Freigabe antibiotischer Wirksubstanz - beispielsweise über den nachfolgenden Zeitraum von zwei bis vier Wochen - ist dann allerdings ein Wachs auf Basis Glykolsäure nicht mehr geeignet. Die Untersuchungen der Anmelderin haben gezeigt, daß sich hier ganz besonders vergleichsweise niedermolekulare Milchsäurewachse durch Einstellung des geforderten Wertstoff-Freigabeprofils auszeichnen. So sind Milchsäureoligomere mit mittleren Oligomerisationsgraden von etwa 6 bis 20 Milchsäureeinheiten pro Molekül und insbesondere etwa 8 bis 15 Milchsäureeinheiten pro Molekül besonders geeignet eine lokale gleichmäßige Antibiotikafreigabe für den Zeitraum von etwa 1 bis 4 Wochen nach Implantation in lebendes Gewebe sicherzustellen. In besonders einfacher Weise können entsprechende Milchsäurewachse für diesen Anwendungsfall unter Mitverwendung von insbesondere mehrfunktionellen Alkoholen im Sinne der genannten deutschen Schutzrechte der Anmelderin hergestellt werden. So können die besonders körperfreundlichen Glycerinester der Milchsäure mit im Mittel 3 bis 5 Milchsäureeinheiten pro Oligoesterrest am Glycerin mit besonderem Vorteil Verwendung finden. Bei dem Einsatz von Ethylenglykol gelten die Angaben zu den mittleren Molekulargewichten der Oligomilchsäurereste sinngemäß.

Untersuchungen über das Abbauverhalten von Oligoestern der hier geschilderten Art haben gezeigt, daß entsprechende Milchsäure-basierte Glycerinwachse eine beträchtliche Resistenz gegen den hydrolytischen Abbau aufweisen können, so daß ihre Aufnahme in den lebenden Organismus wesentlich länger dauern kann als der zeitverzögerte Abschnitt der Freisetzung des pharmakologischen Wertstoffes. Für die Brauchbarkeit dieses Trägermaterials im Sinne der erfindungsgemäßen Aufgabe ist das unerheblich. Wird im Einzelfall dieser retardierte Abbau des Trägermaterials auf Milchsäure/Glycerin-Basis als störend angesehen, so kann durch weitere Bestimmungselemente der Erfindung, die im nachfolgenden noch geschildert werden und insbesondere in einer binären Mischung von Glykolsäure-basierten und Milchsäure-basierten Wachsanteilen liegen, gegengesteuert werden.

Die vergleichsweise niedermolekularen Wachse der hier geschilderten Art und insbesondere solche Wachse auf Milchsäurebasis werden allerdings wichtigen weiteren Anforderungen an die Beschaffenheit eines solchen Depot-Materials nicht gerecht, wobei hier insbesondere Fragen der Handhabbarkeit, der Verklebungsfreiheit, der Lagerbeständigkeit und dergleichen betroffen sind. Für die erfindungsgemäße Lehre in ihrer Gesamtheit ist dieser Mangel der Wachse unbedeutend. Er wird durch die Mitverwendung der zuvor definierten Vernetzerkomponenten in der im nachfolgenden noch geschilderten Weise kompensiert.

Die hier angestellten Überlegungen für die Auswahl und Beschaffenheit bevorzugter Trägermaterialien für die unmittelbare Implantation in lebendes Gewebe können Gültigkeit auch für die Zusammenstellung von Retard-Systemen für die enterale und/oder sonstige topische Applikation besitzen. Begreiflicherweise erweitern sich hier aber die jeweils geeigneten Bereiche möglicher Vertreter für das Trägermaterial im Sinne der Erfindung. Anhand des nachfolgenden Beispiels sei das verdeutlicht: Bei der oralen Applikation ist das Vorliegen freier Carboxylgruppen im Trägermaterial nicht nur tolerierbar, für den Mechanismus der Bioadhäsion ist die Interaktion der Schleimhautbereiche mit funktionellen Carboxylgruppen auf der Oberfläche des verabreichten Mehrstoffgemisches ein bekannter und wichtiger Mechanismus, der zur zeitverlängerten Anbindung des applizierten Mehrstoffgemisches an den jeweiligen Schleimhautbereichen führt. So sind Carboxymethylzellulose und/oder Polymer- oder Copolymerverbindungen der Acrylsäure und/oder Methacrylsäure mit freien Carboxylgruppen wichtige bekannte polymere Träger für bioadhäsive Wirkstoffzubereitungen der hier betroffenen Art.

Erfindungsgemäß kann über eine entsprechende Ausgestaltung des Trägermaterials diesem bekannten Mechanismus zur Schleimhaut-Interaktion beispielsweise dadurch entsprochen werden, daß zur Regulierung des mittleren Oligomerisationsgrades der Hydroxycarbonsäureester einwertige und/oder bevorzugt mehrwertige Carbonsäuren eingesetzt werden. Das entstehende Oligomermolekül ist dann durch endständige Carboxylgruppen gekennzeichnet, die bei der Applikation im Fertigprodukt in die bioadhäsive Interaktion mit den Schleimhautbereichen treten können. Als Molekulargewichts-regelnde Carbonsäuren eignen sich hier insbesondere physiologisch unbedenkliche Vertreter, wobei als Beispiel die sogenannten Genußsäuren zu nennen sind, die bekanntlich den Bereich einwertiger und mehrwertiger Carbonsäuren umfassen.

Zur Frage der Stabilität gegen hydrolytischen Abbau gelten dann auch hier sinngemäß die zuvor angestellten Überlegungen im Zusammenhang mit der Auswahl der bestimmten Hydroxycarbonsäuren zum Aufbau des oligomeren Trägermaterials.

### Die erfindungsgemäß eingesetzten Vernetzerkomponenten

Die im Nachfolgenden als Vernetzerkomponenten beschriebenen Wirkstoffbestandteile sind radikalisch reaktive bevorzugt mehrfunktionelle Komponenten, die über olefinische Doppelbindungen zu Polymerverbindungen umgesetzt werden können. Im einsatzfertigen Depot-Material sind die reaktiven Vernetzer durch Polymerisation und/oder Vernetzung zu den entsprechenden Polymerverbindungen umgewandelt und bestimmen in dieser Form wichtige Stoffeigenschaften des Depot-Materials. Besondere Bedeutung besitzen sie unter anderem für die hinreichende Verfestigung der Gesamtmasse, die als Hauptkomponente die zuvor dargestellten Trägermaterialien auf Basis zäh-viskoser bis wachsartiger Oligomerverbindungen enthalten. Es hat sich überraschenderweise gezeigt, daß schon durch vergleichsweise geringe Mengen solcher Reaktivbestandteile weiche Wachse zu hinreichend festen, nicht klebrigen oder gar tackfreien Werkstoffen führen können. Dabei kann insbesondere die in-situ Aushärtung der reaktiven Vernetzerkomponenten in Abmischung mit dem jeweils gewählten Trägermaterial auf Wachsbasis zu Kombinationsmaterialien führen, die das geforderte anwendungstechnische Gesamtspektrum der Eigenschaften aufweisen. Auch hier schließt das baukastenartige Arbeitsprinzip der Erfindung eine Mehrzahl von konkreten Ausgestaltungen des Fertigproduktes, auf die im nachfolgenden noch eingegangen wird.

Die erfindungsgemäß als Modifizierungsmittel vorgesehenen reaktiven Vernetzer beziehungsweise die daraus durch Abreaktion entstehenden Polymerverbindungen basieren auf olefinisch ungesättigten Estern niederer Hydroxycarbonsäuren mit 2 bis 6 C-Atomen und/oder deren Oligomeren. Auf diese Weise kann auch hier der Abbau der implantierten Polymerverbindungen und die Resorption auch dieses Werkstoffbestandteiles sichergestellt werden.

Für die Verwendung als Vernetzerkomponenten sind insbesondere mehrfunktionelle Verbindungen der angegebenen Art geeignet, die im Molekül mehr als nur eine reaktive olefinische Gruppe aufweisen. Geeignet sind insbesondere 2 bis 4-funktionelle olefinisch reaktive Hydroxycarbonsäurederivate, die einen wenigstens substantiellen Anteil der Vernetzerkomponenten ausmachen sollen.

Bevorzugte Vernetzer im Sinne der erfindungsgemäßen Lehre sind Ester und/oder Oligoester niederer mehrfunktioneller Alkohole oder Carbonsäuren mit Milchsäure und/oder Glykolsäure, die zusätzlich endständige olefinisch ungesättigte Reste aufweisen. Besonders geeignet sind hier wiederum Vernetzerkomponenten, die sich von niederen 2 bis 4-wertigen Alkoholen, insbesondere dem Ethylenglykol und/oder dem Glycerin ableiten und endständig mit Resten der Acrylsäure und/oder der Methacrylsäure - im Rahmen der Erfindungsbeschreibung als (Meth)acrylsäure-Reste bezeichnet - olefinisch substituiert sind.

Reaktivkomponenten der hier geschilderten Art sind beispielsweise eingehend beschrieben in der DE-A1 32 04 504 und der DE-A1 32 29 635. Dargestellt sind dort insbesondere schwer flüchtige, bei Raumtemperatur flüssige bis feste (Meth)-Acrylatverbindungen mit endständigen (Meth)acrylsäure-Resten an einer Oligomerenkette aus Hydroxycarbonsäuren, wobei die Polyester-Oligomeren ein mittleres Molekulargewicht im Bereich von etwa 200 bis 600 besitzen. Die polyfunktionellen (Meth)-acrylsäure-Ester dieser Art sollen dort als chirugische Bindemittelsysteme zum Verkleben von körpereigenem Hartgewebe, gewünschtenfalls zusammen mit Kunststoff und/oder Metall eingesetzt werden. Verbindungen dieser Art sind ein geeignetes Beispiel für Vernetzer im Sinne des erfindungsgemäßen Handelns.

Reaktive Vernetzerkomponenten sind aber nicht an (Meth)-acrylsäure-Ester der geschilderten Art gebunden. Die Einführung von reaktiven olefinischen Gruppierungen in ein Estermolekül der erfindungsgemäß betroffenen Art kann in beliebiger an sich bekannter Weise erfolgen. Lediglich beispielhaft sei hier auf die bekannte Einführung endständiger olefinischer Gruppen an Carboxylgruppen-enthaltenden Grundkörpern verwiesen. Sinngemäß gelten hier dann entsprechende Überlegungen, wie sie bereits im Zusammenhang mit den Trägermaterialien angestellt worden sind, die in einer bevorzugten Ausführungsform Hydroxylgruppen-terminierte Oligomere sind, für bestimmte Anwendungszwecke - beispielsweise für Bioadhäsiv-Materialien - aber als entsprechende Carboxylgruppenterminierte Verbindungen ausgebildet sind. Eine insbesondere für den Einsatz im lebenden menschlichen und tierischen Organismus wichtige Klasse von Vernetzerkomponenten sind Umsetzungsprodukte von Glycerinestern der Milchsäure und/oder von Oligo-Milchsäuren, die mit endständigen (Meth)acrylsäure-Resten modifiziert sind. Neben oder anstelle der Milchsäure können hier auch Glykolsäureeinheiten in das Vernetzermolekül eingebaut sein. In der bevorzugten Ausführungsform enthalten diese Vernetzer im statistischen Mittel wenigstens zwei olefinisch ungesättigte Endgruppen, wobei vollständig mit (Meth)acrylsäure und/oder deren reaktionsfähigen Derivaten abreagierte Vernetzerkomponenten eine besonders interessante Klasse von Vernetzerkomponenten im Sinne der erfindungsgemäßen Lehre sind. Diese im statistischen Mittel 2 bis 3 olefinische Endgruppen/Mol-Glycerin aufweisenden Vernetzer können pro Mol Glycerin im statistischen Mittel in einer wichtigen Ausführungsform 3 bis 12 Mol Milchsäurereste, insbesondere 3 bis 6 Mol Milchsäurereste, enthalten. Reaktive Glycerinderivate im Molverhältnis Glycerin/Milchsäure/(Meth)acrylsäure von etwa 1/4/3 sind hochwirksame Vernetzerkomponenten, die schon in Mischungsverhältnissen von beispielsweise 1 bis 5 Gew.-% - bezogen auf das Gewicht des nicht-reaktiven Trägermaterials - befähigt sind, einen zäh-viskosen oder weichen wachsartigen Träger so in seiner physikalischen Beschaffenheit umzugestalten, daß er den erfindungsgemäßen Anforderungen entspricht.

Insbesondere für das Gebiet der in den lebenden Körper zu implantierenden Retard-Systeme gelten dabei die nachfolgenden Überlegungen: Die erfindungsgemäße Lehre will hier die Möglichkeit schaffen Stoffgemische zur Verfügung zu stellen, die bezüglich aller ihrer Komponenten in Richtung auf Körperverträglichkeit optimiert sind. Besondere Bedeutung kann dieser Überlegung im Zusammenhang mit den Vernetzerkomponenten zukommen.

Die Vernetzer sind radikalisch reaktive Systeme, die bei Herstellung, Transport und/oder Lagerung die Mitverwendung von Radikal-Inhibitoren zum sicheren Ausschluß einer unerwünscht frühzeitigen Abreaktion des Systems benötigen. Solche Radikal-Inhibitoren werden insbesondere auch schon bei der Herstellung der radikalisch reaktiven Verbindungen - beispielsweise also der mehrfunktionellen (Meth)acrylatester - benötigt. Gerade die hohe Vergelungstendenz mehrfunktioneller (Meth)acrylsäure-Ester der hier betroffenen Art fordert den Einsatz stark wirksamer Inhibitoren auch schon bei der Herstellung. Nach einem wesentlichen Element der Erfindung sind die mit eingesetzten Vernetzerkomponenten frei von - nach Art und/oder Menge - unerwünschten Inhibitoren aus der Herstellung, der Lagerung und/oder der Verarbeitung der mehrfunktionellen Komponenten.

Ein besonders wichtiges Beispiel für einen geeigneten Inhibitor im Sinne des erfindungsgemäßen Handelns sind Tocopherolverbindungen und hier insbesondere das Alpha-Tocopherol und damit das Vitamin E.

Der Einsatz von physiologisch verträglichen Tocopherolverbindungen und insbesondere Vitamin E als Applikationsinhibitor, gegebenenfalls aber auch schon als Herstellungsinhibitor im Zusammenhang mit mehrfunktionellen (Meth)acrylsäureverbindungen, ist im einzelnen Gegenstand der älteren Anmeldung DE 39 39 161 der Anmelderin, die Offenbarung dieses älteren Schutzrechtes wird hiermit ausdrücklich auch zum Gegenstand der Erfindungsoffenbarung gemacht.

Bedeutungsvoll kann für die Vernetzerkomponenten im Sinne der erfindungsgemäßen Lehre auch der Anteil der Offenbarung aus der genannten älteren Anmeldung DE 39 39 161 sein, der den Einsatz von olefinisch reaktiven (Meth)acrylsäure-Estern beschreibt, die frei sind von Lösungsmitteln beziehungsweise insbesondere herstellungsbedingten Lösungsmittelresten. Dementsprechend wird in der bevorzugten Ausführungsform auch erfindungsgemäß bei der Herstellung der Vernetzerkomponenten auf die Mitverwendung von insbesondere physiologisch bedenklichen Lösungsmitteln von vornherein verzichtet. Verwiesen wird in diesem Zusammenhang gleichzeitig auf die Offenbarung der auf die Anmelderin zurückgehenden Druckschriften gemäß DE-A1 38 43 854, 38 43 938, 38 43 930 und 38 43 843.

Die Mischungsverhältnisse zwischen dem Trägermaterial einerseits und den Vernetzerkomponenten beziehungsweise den daraus durch Abreaktion entstehenden Polymerverbindungen können in weiten Bereichen schwanken. Sie werden im einzelnen durch eine Mehrzahl von Parametern bestimmt, von denen hier insbesondere genannt seien: Beschaffenheit des Trägermaterials, insbesondere seine physikalische Beschaffenheit bei Raumtemperatur und bei Anwendungstemperatur, Anforderungsprofil an die Beschaffenheit des Depot-Materials bei Lagerung und Applikation, Reaktivität und Eigenbeschaffenheit der Vernetzerkomponenten beziehungsweise der durch Abreaktion daraus entstehenden Polymerverbindungen, sowie Anforderungsprofil aus dem beabsichtigten Einsatzzweck an das Gesamtmaterial. Es leuchtet insbesondere zum letzten Gesichtspunkt sofort ein, daß Pharmaka-Release-Materialien für die Implantation in den lebenden Körper durchaus andere Forderungen stellen können als beispielsweise ein Depot-Material für die verzögerte Freigabe biologischer Wirkstoffe beispielsweise bei derem Einsatz im landwirtschaftlichen Bereich. Zu den Mischungsverhältnissen von nicht-reaktivem Trägermaterial einerseits und den reaktiven Trägerkomponenten andererseits wird im allgemeinen gelten, daß die Vernetzerkomponenten nicht mehr als etwa die Hälfte des Gesamtsystems ausbilden. In bevorzugten Ausführungsformen werden deutlich geringere Mengen an Vernetzerkomponenten eingesetzt. So gilt hier insbesondere, daß die modifizierenden und wenigstens anteilsweise abreagierten Vernetzerkomponenten in Mischungsverhältnissen von etwa 0,5 bis 40 Gew.-% und vorzugsweise in Mischungsverhältnissen von etwa 1 bis 20 Gew.-% Vernetzer - Gew.-% hier bezogen auf die Summe von Trägermaterial und Vernetzerkomponenten - vorliegen können. Für den Einsatz der erfindungsgemäßen Retard-Systeme im medizinischen Bereich und insbesondere für die Implantation in lebendes Gewebe können Anteile der Vernetzerkomponenten von höchstens etwa 10 Gew.-% bevorzugt sein. Wie bereits angegeben werden hier mit Mischungsverhältnissen bis höchstens etwa 5 Gew.-% und insbesondere mit Abmischungen gute Ergebnisse erhalten, die etwa 2 bis 5 Gew.-% an Vernetzerkomponenten enthalten. Gültigkeit hat das insbesondere für die Verwendung von vergleichsweise stark vernetzenden Verbindungen beispielsweise von der Art der 3-funktionellen (Meth)acrylsäure-Ester der Hydroxyl-terminierten Glycerin/Milchsäure-Ester beziehungsweise Oligo-Ester.

### Die verschiedenen Ausführungsformen der Retard-Systeme

Bereits einleitend ist auf die Schwierigkeiten verwiesen worden, sichere Aussagen über das Freigabeverhalten von Retard-System zu machen, da eine Vielzahl von Interaktionsparametern in die letztlich auftretende Wirkung des Systems eingehen. Es ist ein besonderer Vorteil der erfindungsgemäßen Lehre, daß hier die Möglichkeit gegeben wird, durch Variation in der konkreten Ausgestaltung des Systems eine Optimierung in Richtung auf das jeweils im Einzelfall vorgegebene Anforderungsprofil vornehmen zu können. Verständlich wird das aus den nachfolgenden Überlegungen:
Die Hauptmasse oder wenigstens ein wesentlicher Anteil des Depot-Materials wird in der Regel durch das zuvor geschilderte Trägermaterial gebildet. Durch Auswahl der die Oligomer- beziehungsweise Polymerverbindungen-bildenden Komponenten und die sich daraus ableitenden Freigabecharakteristika kann Einfluß auf die Zeitsteuerung des Freigabemechanismus genommen werden. Das gilt bereits für einheitliche, in der Konstitution bestimmt ausgewählte und aufgebaute Verbindungstypen dieser Art. Eine weitere Modifikation erschließt sich durch den Einsatz wenigstens binär aufgebauter Systeme im Trägermaterial, die unterschiedliche Wachs- beziehungsweise Harztypen miteinander vereinigen. Hier werden also beispielsweise vergleichsweise schnell abbauende Oligomer- beziehungsweise Polymerverbindungen einerseits mit vergleichsweise langsamer abbauenden Verbindungen dieser Art in Mischung miteinander eingesetzt. Eine weitere Einflußmöglichkeit ist über die bestimmte Art und Weise der Vermischung solcher unterschiedlicher Komponenten des Trägermaterials miteinander gegeben. Es erschließt sich der gesamte Bereich von homogen Mischungen bis zu Mischungstypen, die abgegrenzte Materialbereiche ausgewählter bestimmter Zusammensetzung mit entsprechend abgegrenzten Materialbereichen einer anderen Zusammensetzung und dementsprechend eines anderen Abbauverhaltens verbinden. In der hier dargestellten Form kann beispielsweise Einfluß genommen werden auf eine sich im praktischen Einsatz ausbildende Porosität beziehungsweise Durchlässigkeit eines Blockes aus Trägermaterial-Komposition gegenüber Durchtritt von Körperflüssigkeit und/oder Durchtritt von Zellwachstum. Schließlich kann auch eine schichtartige Anordnung der verschiedenen Komponenten des Trägermaterials zu einem primär geschlossenen Materialblock in Betracht kommen, der bestimmten Anforderungscharakteristiken im praktischen Einsatz durch die unterschiedliche Abbaugeschwindigkeit der einzelnen schichtartigen Blöcke entspricht.

Die hier angedeuteten und auf die jeweilige Modifikation des Trägermaterials zurückzuführenden Modifikationen werden durch die erfindungsgemäße Mitverwendung der Vernetzerkomponenten substantiell erweitert. Sinngemäß gelten die zuvor gemachten Angaben auch für die Stoffgemische aus Trägermaterial und Vernetzerkomponenten. So können Trägermaterial und Vernetzerkomponenten - zusammen mit den Wertstoffen - in einer Ausführungsform in weitgehend homogener Abmischung miteinander vorliegen. Ebenso ist es aber auch möglich, daß die Vernetzerkomponenten beziehungsweise die daraus entstandenen Polymerverbindungen in zum Beispiel schichtförmiger Anordnung im Retard-System vorliegen, so daß wenigstens weitgehend Vernetzer-freie Bereiche des Wertstoff-Trägergemisches von Materialbereichen mit einem Gehalt an abreagierten Vernetzerkomponenten durchsetzt und/oder umhüllt sind. Vernetzerkomponenten müssen nicht mit allen Trägermaterialien kombiniert sein. So können beispielsweise als Freigabeverzögernde Träger besonders solche Milchsäure-Oligomertypen geeignet sein, die bei Raum- und/oder Anwendungstemperatur zäh-viskose Beschaffenheit haben und beispielsweise aus Gründen der verbesserten Handhabbarkeit mit Vernetzerkomponenten im Sinne der Erfindung durchsetzt und formstabil fixiert werden. Kombinationsmaterialien dieser Art können dann mit weiteren Trägermaterialien - beispielsweise auf Basis von Polymertypen höheren Molgewichtes oder den ohnehin in festerer Form anfallenden Glykolsäure-Oligomeren beziehungsweise -Polymeren vermischt werden.

Die hier aufgezählten Möglichkeiten machen das Folgende klar: Durch die erfindungsgemäß eingesetzten Vernetzerkomponenten und die damit mögliche Beeinflussung der Stoffbeschaffenheit derjenigen Bereiche des Depot-Materials, das durch die Vernetzerkomponenten beziehungsweise die daraus entstandenen Polymerbindungen geprägt ist, wird erstmalig möglich insbesondere den Bereich vergleichsweise niedermolekularer Trägermaterialien der hier betroffenen Art von Hydroxycarbonsäure-Oligomeren dem angestrebten Verwendungszweck zuzuführen, wobei für wichtige Anwendungszwecke gerade diesen Trägermaterialien vergleichsweise niederen Molekulargewichts die in vielen Fällen gewünschte verzögerte Freigabecharakteristik zukommt. Behinderungen des regenerierenden Zellwachstums können dadurch zuverlässig ausgeschlossen werden, daß die individuellen Bereiche der entsprechenden Trägermaterialien - beispielsweise auf Milchsäurebasis - klein gehalten werden. Dabei kann das erfindungsgemäß stabilisierte Depot-Material als feinkörniges Gut oder gar als Pulver in die betroffenen Gewebebereiche eingetragen oder in topischen Applikationsformen eingesetzt werden, ebenso ist es aber möglich größere Raumformen beispielsweise in Form von Platten, Stäben, Kugeln oder in beliebiger anderer Raumform im lebenden Gewebe zu fixieren und durch die konkrete Zerfallscharakteristik einer solchen größeren Raumeinheit im lebenden Organismus unerwünschte Verzögerungen des regenerierenden Zellwachstums mit Sicherheit auszuschließen.

### Die Aushärtung der Vernetzerkomponenten

Gerade im Zusammenhang mit den zuletzt diskutierten Überlegungen zur möglichen Vielgestaltigkeit der gemäß der Erfindung auszugestaltenden Depot-Materialien wird verständlich, daß auch für die Überführung der erfindungsgemäß definierten Vernetzerkomponenten in ihre Polymerverbindungen eine Vielzahl von Möglichkeiten besteht. Damit ist eine Anpassung an die jeweiligen Gegebenheiten möglich und zuverlässig die Hauptaufgabe zu erfüllen ein Depot-Material zur Verfügung zu stellen, das den Wertstoff im praktischen Einsatz in unversehrtem Zustand zur Verfügung stellt.

Untersuchungen der Anmelderin haben gezeigt, daß in einem doch offenbar breiten Bereich konkreter Träger/Wertstoff-Gemische die homogene Einmischung von Vernetzerkomponenten und nachfolgende in-situ Abreaktion möglich ist, ohne damit eine substantielle Wertstoffschädigung oder Wertstoffminderung auszulösen. In dieser einfachsten Ausführungsform der erfindungsgemäßen Materialien sind die modifizierenden Polymerverbindungen dann aus den radikalisch reakiven Vernetzerkomponenten in Abmischung mit wenigstens einem Anteil des Depot-Materials auf Basis Träger/Wertstoff in-situ hergestellt worden. So können Wirkstoffgemische von Glycerin/Milchsäure-basierten Oligomeren als Trägermaterial und Aminoglykosid-Antibiotika durch Aufschmelzen des Trägermaterials bei vergleichsweise niederen Temperaturen und homogenes Einmischen der Wertstoffe hergestellt werden. Zum Aufschmelzen der genannten Trägermaterialien sind beispielsweise Temperaturen unterhalb oder höchstens im Bereich von etwa 70°C durchaus ausreichend. Innerhalb des gleichen Temperaturbereichs können geringe Mengen, beispielsweise 1 bis 5 insbesondere 2 bis 4 Gew.-% an Vernetzerkomponenten auf Basis Glycerin/-Milchsäure/(Meth)acrylsäure homogen eingemischt werden. Durch nachfolgende radikalische Abreaktion gelingt die in-situ Vernetzung der Vernetzerkomponenten zu den entsprechenden Polymerverbindungen, die dann das endgültige Erscheinungsbild des Pharmakadepot-Materials entscheidend mitbestimmen. Es können beispielsweise im Trockenzustand weitgehend tack-freie plattenförmige Formkörper hergestellt werden, die gewünschtenfalls einer weiteren Zerkleinerung unterworfen werden können.

Zeigt sich, daß zwischen dem jeweiligen Wertstoff und den radikalisch reaktiven Systemen der erfindungsgemäß mitverwendeten Vernetzerkomponenten unerwünschte, die Wertstoffcharakteristik mindernde Interaktionen zu befürchten sind, läßt sich das erfindungsgemäße Prinzip gleichwohl mühelos anwenden: In einem solchen Fall werden Träger/Wertstoff-Gemische in eine vorbestimmte partikuläre Form gebracht und dann mit einer Wertstoff-freien Umhüllung mit einem Gehalt an Vernetzerkomponenten im Sinne der erfindungsgemäßen Definition versehen. Auch hier können wieder Gemische von Trägermaterial und Vernetzerkomponenten eingesetzt werden, die jetzt jedoch im Bereich der Umhüllung Wertstofffrei sind. Dabei kann das Trägermaterial gleich oder verschieden sein von dem im Innenbereich eingesetzten Trägermaterial für den freizusetzenden Wertstoff. In der nachfolgenden Aushärtung kann eine substantielle Interaktion der Vernetzerkomponenten mit dem im Kern eingebetteten Wertstoff nicht stattfinden. Die hier dargestellten zwei Ausführungsformen sind lediglich beispielhaft zu verstehen. Sie können getrennt voneinander oder auch in Kombination miteinander eingesetzt werden, wobei sich über die zuvor geschilderten zahlreichen Kombinationsmöglichkeiten für die konkrete Ausgestaltung des jeweiligen Depot-Materials sinngemäß entsprechende weitere konkrete Ausführungsformen ableiten.

In einer bevorzugten Ausführungsform für die erfindungsgemäße Lehre kann die Reaktionsauslösung unter Bedingungen durchgeführt werden, die den Eintrag physiologisch unerwünschter Komponenten in das Fertigprodukt ausschließen. Verwiesen wird hierzu auf die Angaben der älteren deutschen Patentanmeldung DE-A-40 37 516 der Anmelderin, deren einschlägige Offenbarung hiermit ebenfalls ausdrücklich zum Gegenstand der vorliegenden Erfindungsoffenbarung gemacht wird.

Geschildert sind in dieser älteren Anmeldung DE-A-40 37 516 hochfeste und abbaubare Wertstoffe und Formkörper für die Implantation in den menschlichen und tierischen Organismus, die auf Basis gehärteter (Meth)acrylsäure-Ester von mehrfunktionell Hydroxyl-terminierten Oligomeren niederer Hydroxycarbonsäuren aufgebaut sind. Sie kennzeichnen sich unter anderem dadurch, daß sie von Reaktionshilfsmitteln und/oder sonstigen Zusatzstoffen aus der Herstellung (Wirkstoffverunreinigungen) im wesentlichen frei sind, die nach Art und/oder Menge physiologisch unerwünscht sind, wobei hier insbesondere im Rahmen ihrer Herstellung eine Abstimmung - jeweils nach Art und Menge - zwischen den Inhibitoren der mehrfunktionellen (Meth)acrylsäure-Ester und den zu ihrer Vernetzung gegebenenfalls eingesetzten Initiatoren beziehungsweise Startersysteme vorgenommen worden ist. Die Härtung soll entweder durch Strahlenhärtung - insbesondere UV-Strahlung - erfolgen oder mit Startersystemen vorgenommen werden, die im wesentlichen auf körperverträglichen Komponenten beruhen oder aber wenigstens nach Art und Menge so eingeschränkt sind, daß die beim Abbau frei werdenen Reststoffe keine physiologischen Probleme auslösen.

Im Rahmen der Strahlungshärtung kommt neben der bereits genannten UV-Lichthärtung auch der Einsatz anderer reaktionsauslösender Strahlungstypen in Betracht, wobei genannt seien zum Beispiel Laserstrahlen, Röntgenstrahlen oder Gammastrahlen. Die Mitverwendung von an sich bekannten Initiatoren ist möglich, verwiesen wird in diesem Zusammenhang beispielsweise auf die druckschriftlichen Angaben in Enzycl. Polym. Sci. and Eng. (2. Auflage) Band 11, 187 - 207 und einschlägige Handelsprodukte, beispielsweise der Firmen Merck, Darmstadt (DE) und Ciba-Geigy, Schweiz.

Möglich ist erfindungsgemäß insbesondere aber auch die Härtung des Reaktivmaterials in an sich bekannter Weise durch chemisch initiierte radikalische Polymerisation. Für den Einsatz hier bekannter Hilfsmittel ergibt sich die nachfolgende Erleichterung bei der Auswahl der Reaktionshilfsmittel: Der Stand der Technik kennt eine Vielzahl von Redox-Systemen auf Basis peroxidischer Verbindungen, die in Kombination mit Reduktionsmitteln und/oder Metallverbindungen solcher Metalle eingesetzt werden, die in mehreren Wertigkeitsstufen auftreten können. Verwiesen wird in diesem Zusammenhang beispielsweise auf die zusammenfassende Darstellung in PROGRESS IN POLYMER SCIENCE, Vol.8, Pergamon Press, Oxford New York, 1982, S. 61 - 131 und die umfangreiche darin zitierte Primärliteratur.

Bei der Auswahl geeigneter Redox-Systeme, die insbesondere nach Art und Menge der im abreagierten Material zurückbleibenden Reststoffe keine wesentlichen physiologischen Bedenken auslösen, kommt dem Handeln im Rahmen der Erfindung der folgende Tatbestand besonders zugute: Physiologisch vergleichsweise unbedenkliche Reaktantensysteme sind nach dem Stand der Technik insbesondere beschrieben im Zusammenhang mit wäßrigen Polymerisationssystemen. Das erfindungsgemäß zu vernetzende Material ist an sich ein wasserfreies System. Aufgrund seiner oligomeren Struktur, die auf niedere Hydroxycarbonsäuren zurückgeht, sind jedoch die Löslichkeitsverhältnisse auch im Rahmen der Erfindung für solche Komponenten häufig sichergestellt, wie sie sonst in den wäßrigen Systemen zum Einsatz kommen. Es wird damit möglich, eine wirklich homogene Verteilung physiologisch weitgehend unbedenklicher Aktivatorsysteme auch in der wasserfreien Reaktionsphase der mehrfunktionellen (Meth)acrylsäure-Ester beziehungweise ihrer Abmischungen mit den Trägermaterialien zu erreichen, um damit dann bei vorgegebenen Temperaturen die Vernetzung zu bewirken.

Aus der großen Klasse geeigneter Komponenten für solche Redox-Systeme seien hier lediglich beispielhaft genannt: Peroxidverbindungen wie Persäuren, Diacylperoxide, Hydroperoxide und dergleichen, wobei physiologisch verträglichen Säuren, beispielsweise sogenannte Genußsäuren, als Peroxid-bildendem Bestandteil besondere Bedeutung zukommen kann. Aus der großen Klasse der Aktivatoren beziehungsweise Reduktionsmittel bieten sich Verbindungen an wie Ascorbinsäure, Sorbose, Fructose, Dextrose oder andere Zucker, bei denen es sich durchweg um physiologisch unbedenkliche Komponenten handelt.

Zur Stimulierung beziehungsweise Aktivierung der Redoxreaktion geeignete Metallverbindungen leiten sich insbesondere vom Eisen ab, das in Form 2- und/oder 3-wertigen Eisens in an sich bekannter Weise den Redox-Systemen zugegeben werden kann. Die homogene Einmischung des Eisens gelingt beispielsweise besonders sicher mit den entsprechenden Salzen der Glykolsäure und/oder der Milchsäure.

Wie bereits angegeben und aus der Vielgestaltigkeit der möglichen konkreten Ausführungsformen für die Retard-Systeme im Sinne der Erfindung einleuchtend, ist eine nahezu unbegrenzte Anwendbarkeit des Träger/Vernetzer-Systems auf den breiten Bereich interessanter Wertstoffe medizinischer und allgemein biologischer Wirksamkeit gewährleistet. Nicht nur die Wirkstoffe selber, auch ihre Anwendungsformen können in breites Rahmen variiert werden. Lediglich beispielhaft sei das wieder an erfindungsgemäß formulierten Wertstoffgemischen mit antibiotischer Wirkung erläutert: Das Pharmakadepot-Material kann beispielsweise im Rahmen operativer Eingriffe in die infektionsgefährdeten Gewebebereiche eingetragen - beispielsweise eingelegt - und darin verschlossen werden. Andererseits ist es aber auch möglich, daß Depot-Material auf offene Wundbereiche, zum Beispiel Verbrennungswunden und dergleichen, aufzutragen und dort zu belassen und/oder im Zuge des Verbandswechsels wenigstens anteilsweise abzutragen und durch neues Pharmakadepot-Material zu ersetzen.

Die Wertstoffkonzentrationen im Pharmakadepot-Material können in weiten Bereichen schwanken. Der übergeordnete Gedanke für die in der Praxis wichtigen Stoffmischungen ist die Einstellung der gewünschten zeitverzögerten Wirkstofffreigabe. Im allgemeinen wird der Wertstoff nicht mehr als etwa 40 bis 45 Gew.-% des Retard-Systems in seiner Gesamtheit ausmachen. Für das wichtige Gebiet der medizinischen Wertstoffe in ihrer Anwendung auf den menschlichen und den tierischen Körper werden Wertstoffkonzentrationen von etwa 20 bis 25 Gew.-% und vorzugsweise von etwa 10 bis 15 Gew.-% selten überschritten werden. Hochwirksame Wertstoffe wie die bereits erwähnten antibiotischen Wirksubstanzen, Zytostatika, Hormone und dergleichen werden im allgemeinen in Mengen von etwa 0,1 bis 10 Gew.-%, insbesondere in Mengen von etwa 1 bis 8 Gew.-% in den Pharmaka-Release-Materialien verwendet werden.

Die Wertstoffe werden im allgemeinen mit wenigstens einem Anteil des eingesetzten Trägermaterials in inniger Abmischung vorgelegt werden, um zu einer optimalen Steuerung der Wertstofffreisetzung zu kommen. Im übrigen gelten aber die zuvor gebrachten Überlegungen zur Möglichkeit der inhomogenen Wertstoffverteilung in der jeweiligen Einheit des Retard-Systems. Die Applikationsform dieser Retard-Systeme im Sinne der erfindungsgemäßen Lehre reicht von pulverförmiger Beschaffenheit zu beliebig ausgebildeten Raumkörpern. Die quantitativ leicht erfassbare Menge der applizierten Retard-Systeme und die pro Raum- beziehungsweise Gewichtseinheit dieses Retard-Systems vorgegebene Menge des jeweiligen Wertstoffs ermöglicht die quantifizierte Verarbreichung der im Depot-Material vorliegenden Wertstoffe.

Der Einsatz der erfindungsgemäßen Lehre im Zusammenhang mit weniger kritischen Anbietungsformen als der direkten Implantation in lebendes Gewebe ermöglicht die Mitverwendung üblicher galenischer Hilfsstoffe und/oder die Zubereitung der erfindungsgemäßen Retard-Systeme praxisüblicher Einsatzformen. Wiederum beispielhaft wird das aus folgendem verständlich: Erfindungsgemäß ausgebildete Pharmaka-Depot-Materialien können in feinpartikulärer Form zu Tabletten, Zäpfchen, Salben und dergleichen verarbeitet sein und in dieser Form in den Körper eingebracht oder auf die Körperoberfläche aufgetragen werden. Diese galenischen Hilfsstoffe können ihrerseits nicht-redartierte Pharmakaanteile enthalten, die beim Ein- beziehungsweise Auftrag sofort wirksam werden, woraufhin sich der erfindungsgemäß zeitverzögerte Wirkungsabschnitt der retardierten Wirkstofffreisetzung anschließt.

In anderen Fällen können an sich bekannte Hilfsstoffe mitverwendet werden, um gezielt Effekte einzustellen beziehungsweise zu verstärken, die auch charakteristisch für die im Sinne der Erfindung konkret eingestellten Stoffeigenschaften sind. Am Beispiel bioadhäsiver Wertstoffzubereitungen sei das erläutert: Die kovalente Bindung der Oberfläche des Mehrstoffgemisches im Sinne der erfindungsgemäßen Lehre mit der Schleimhautoberfläche und damit der Effekt der Bioadhäsion wird unmittelbar durch funktionelle Gruppen im erfindungsgemäßen Trägermaterial (Carboxylgruppen und/oder Hydroxylgruppen) bewirkt. Dieser Effekt kann dadurch weiter ausgebaut werden, daß an sich bekannte Schleimhaut-adhäsive Materialien als Füllstoffe im Mehrstoffgemisch mitverwendet werden. Geeignete und in der Praxis erprobte Materialien sind beispielsweise neben den bereits benannten Carboxymethylzellulosen und (Meth)acrylsäure-Polymerisaten beziehungsweise -Copolymerisaten, Alginate, Hydroxyalkylzellulosen, Gelatine, Pektin, Polyvinylpyrrolidon, feste Polyethylenglykole und dergleichen. Verwiesen sei in diesem Zusammenhang beispielsweise auf die folgenden zusätzlichen Literaturstellen J.D. Smart et al. "An in-vitro investigation of mucosa-adhesive materials for use in controlled drug delivery", J. Pharm. Pharmacol. 1984, 36, 295 - 299 sowie Kinam Park et al. "Bioadhesive polymers as platforms for oral-controlled drug delivery: method to study bioadhesion" International Journal of Pharmaceutics, 19 (1984), 102 - 127.

Die Mengenanteile solcher mitverwendeter Hilfsstoffe können in weiten Bereichen schwanken und können dabei in der konkreten galenischen Ausgestaltung auch ein Mehrfaches der Stoffgemische im Sinne der erfindungsgemäßen Definition ausmachen. Wesentlich ist für diese Präparate dann aber immer, daß ein nicht unbeträchtlicher Wirkungsabschnitt im praktischen Einsatz durch die erfindungsgemäß definierte Stoffkombination bestimmt ist.

### Beispiel 1

### 1. Herstellung und Beschreibung der resorbierbaren Wachse

a. Wachse, hergestellt aus Glycolsäure/Glycerin
Allgemeine Vorschrift zur Herstellung der Umsetzungsprodukte von Glycolsäure mit Glycerin
In einem Dreihalskolben mit Rührer und Destillationsbrücke werden Glycolsäure und Glycerin vorgelegt. Unter vollständiger Inertisierung mit Stickstoff wird auf 150°C aufgeheizt und die Reaktion innerhalb von 3 bis 5 h so lange weitergeführt, bis sich kein Reaktionswasser mehr abspaltet. Dann wird bei 150°C vorsichtig auf 10 Torr evakuiert. Nach weiteren 2 h bei diesen Reaktionsbedingungen wird auf 100°C abgekühlt, das Vakuum aufgehoben, wie zuvor beschrieben neutralisiert und das Produkt noch heiß abgefüllt.
Die Zusammensetzung der Ansätze und die Oligomereigenschaften sind der Tabelle 1 zu entnehmen.

**Tabelle 1**

| Oligohydroxycarbonsäuren aus Glycolsäure und Glycerin | | | | |
|---|---|---|---|---|
| Beispiel | **Edukte** | | Ausbeute Reaktionswasser % | Konsistenz bei Raumtemperatur |
| | Glycolsäure mol | Glycerin mol | | |
| A | 8 | 1 | 100 | trübe, hochviskos |
| | | nach Neutralisation | | trübe, hochviskos |
| B | 9 | 1 | 99,1 | trübe, hochviskos |
| | | nach Neutralisation | | trübe, hochviskos |
| Beispiel | | Viskosität/100°C MK-D bei 20 UpM | | Fadenziehen ( in cm bis zum Abriß) |
| A | | 5000 mPas | | 2 |
| nach Neutralisation | | 5000 mPas | | 7 |
| B | | 10000 mPas | | 2 |
| nach Neutralisation | | 10000 mPas | | 7 |

b. Wachse, hergestellt aus Lactid und Glycerin
Allgemeine Vorschrift zur Herstellung der Umsetzungsprodukte von Lactid mit Glycerin
Lactid (L(-)-Lactid N, der Firma Böhringer Ingelheim) und Glycerin wurden in einer üblichen Laborapparatur unter Stickstoff und unter Rühren innerhalb von einer Stunde auf 195°C erwärmt. Man ließ dann 3 Stunden bei 195°C reagieren und füllte nach Neutralisation heiß ab. Als Katalysator war eine Sn-ll-Chlorid-Lösung in Ether zugesetzt (7 ml einer Lösung von 2,5 g SnCl₂ in 1000 ml Ether bei der Umsetzung von 3 mol Lactid mit einem mol Glycerin).
Die Zusammensetzung und die Oligomereigenschaften sind in Tabelle 2 angegeben.

**Tabelle 2**

| Oligohydroxycarbonsäuren aus Glycerin und Lactid | | | | | |
|---|---|---|---|---|---|
| Beispiel | **Edukte** | | Konsistenz bei Raumtemperat. | Viskosität/100°C MK-D bei 20 UpM | Fadenziehen (in cm bis zum Abriß) |
| | Glycerin mol | Lactid mol | | | |
| C | 1 | 5 | klar,viskos | 8000 mPas | ca. 60 |
| | | nach Neutralisat. | trübe,hochviskos | 10000 mPas | 30 |
| D | 1 | 6 | klar,viskos | 10000 mPas | ca. 50 |
| | | nach Neutralisat. | trübe, hochviskos | 10000 mPas | 30 |

### 2. Herstellung der reaktiven Vernetzerkomponente

2.1 Herstellung des Oligomeren Glykolsäure/Ethylenolykol 4 : 1
In einem 25-Liter-Versuchsreaktor wurden 16,72 kg Glykolsäure und 3,41 kg Ethylenglykol vorgelegt. Nach der Inertisierung wurde der Kristallbrei unter Stickstoff-Strom geschmolzen und dann weiter bis maximal 145 bis 150°C (Sumpftemperatur) aufgeheizt. Nachdem die Reaktion durch Abdestillation von Wasser eingesetzt hatte, wurde sie 11 Stunden lang weitergeführt, bis sich kein Reaktionswasser mehr abspaltete (Brüdentemperaturabfall auf 70 bis 73°C bei einem Reaktionsumsatz von 70 %). Von der abdestillierten wäßrigen Lösung wurde die Menge des Destillats, die Säurezahl (Gehalt an Glykolsäure) und der Wassergehalt nach Karl-Fischer bestimmt.
Um die Reaktion vollständig durchzuführen, wurde bei 150°C vorsichtig auf 400 Torr evakuiert und innerhalb von 2 Stunden der Druck weiter bis auf 10 Torr reduziert und 1 Stunde gehalten. Das Reaktionswasser wurde zur Umsatzbestimmung festgestellt.
Überschüssiges Kondensat wurde in einer Kühlfalle (gekühlt mit Trockeneis und Ethanol) aufgefangen und im Anschluß ebenfalls gewogen. Nach der gesamten Reaktionszeit wird auf 100°C abgekühlt, das Vakuum mit Stickstoff aufgehoben und das Produkt noch heiß abgefüllt. Das Produkt wurde nicht weiter gereinigt sondern direkt zur Herstellung von Oligoglykolsäurebismethacrylat eingesetzt.
2.2 Ausbeute und Massenbilanz

| | |
|---|---|
| Menge Destillat | 4222,8 g |
| Glykolsäure (aus SZ = 46): 263,58 g -Ethylenglykolbestimmung aus HPLC | 263,6 g |

Wassermenge (aus Wasserbestimmung nach Karl-Fischer) = 91,6 % im Destillat = 3868,1 g entsprechen 97,7 % Umsatz
Analysenergebnisse vom Oligomeren

| Bezeichnung | direkt nach Herstellung | 1 Monat alt | 1 Jahr alt |
|---|---|---|---|
| Ansatzgröße | 20 kg | 4,5 kg | 2 kg |
| Konsistenz | pastös | pastös | pastös |
| Viskosität/b.RT (Epprecht-Viskosimeter MK4) | 12500 mPas | 13000 mPas | 12800 mPas |

| Molgewicht: | | | |
|---|---|---|---|
| Mn* | 438 | 455 | 454 |
| Mw | 515 | 533 | 530 |
| %-freie-Glykolsäure | 2,1% | 1,4% | 1,9% |
| %-freies Ethylenglykol | 0,2% | 0,2% | 0,2% |
| Verseifungszahl | 765,4 | 754,4 | 754,0 |

| Verhalten im Wasser: | | | |
|---|---|---|---|
| pH-Wert nach | | | |
| 2 min. | 3,8 | 3,8 | 3,8 |
| 60 min. | 3,4 | 3,4 | 3,4 |
| Peroxid-Gehalt | negativ | | |
| analytische Zusammensetzung des Produktes Glykolsäure/Ethylenglykol | 3,975 : 1 | 3,972 : 1 | 3,972 : 1 |

| | | | |
|---|---|---|---|
| * Bestimmung des Molgewichtes als GPC-Analyse. Da die Eichung mit Polyethylenglykol als Standard durchgeführt wurde, erklärt sich die Differenz zwischen Mn theor. und Mn lt. Molgewichtsbestimmung durch die Eichmethode. | | | |

2.3 Oligoglykolsäure-bismethacrylat
Handelsübliche Methacrylsäure (Fa. Roehm) wird nach der folgenden Vorschrift neu mit Vitamin E inhibiert:
In einer Vakuumdestillationsapparatur werden 15 mol (=1291,35 g) Methacrylsäure (kp. 163°C mit 3,87 g (= 3000 ppm) Phenothiazin (als Stabilisator) versetzt und unter starkem Luftstrom im Wasserstrahlvakuum überdestilliert. In die Vorlage werden 100 ppm Vitamin E (Covitol F-1000-2, 67 %ig, Henkel KGaA) (= 139 mg/l) vorgelegt und unter Rühren die Methacrylsäure überdestilliert. Die Destillation ist beendet, wenn von der Methacrylsäure 932 g überdestilliert sind.
2.4 Reaktionsverlauf
In einem Dreihalskolben mit Rührer und Destillationsbrücke wurden 294 g Oligoglykolsäure, 206,4 g Methacrylsäure sowie 17,5 g p-Toluolsulfonsäure eingewogen und mit 0,86 g Vitamin E (Alpha-Tocopherol) inhibiert. Während der gesamten Reaktion wurde Luft mit einer Geschwindigkeit von mindestens 40 l/h durch das Reaktionsgefäß geleitet.
Durch Abtrennung des entstandenen Wassers bei einer Maximaltemperatur von 105°C wurde die Veresterung durchgeführt bis die Menge an abgetrenntem Wasser über 35,78 g (mehr als 97 % Umsatz) liegt.
Die Destillationsvorlage wurde während der gesamten Reaktionsführung mit Trockeneis/Ethanolgemisch gekühlt. Bei einer maximalen Sumpftemperatur von 105°C und bei 500 mbar betrug die Veresterungszeit zwischen 12 und 14,5 Stunden bei einem Gesamtumsatz von 97 bis 98,5 %. Von der abdestillierten wäßrigen Lösung (aus Kühlfalle und Vorlage) wurde zur Umsatzbestimmung alle 1,5 Stunden die Menge des Destillats, die Säurezahl (Gehalt an Methacrylsäure) und der Wassergehalt nach Karl-Fischer bestimmt.
Über eine Differenzberechnung wird der Wassergehalt und die überdestillierte Menge an Methacrylsäure berechnet, wobei nach jeder Bestimmung überprüft wurde, ob nach genügend Methacrylsäure für die Reaktion zur Verfügung stand. Meistens mußte nach ca. 7,5 bis 8 Stunden noch zusätzlich 0,1 mol Methacrylsäure nachdosiert werden.
Nach beeindeter Reaktion (Umsatz über 97 %) wurde das Produkt bis zur Reinigung abgefüllt.
2.5 Aufarbeitung des Reaktionsproduktes
Das Reaktionsprodukt ist zum Ende der Reaktionszeit nicht ganz säurefrei. Deshalb wurde es mit Ca(OH)₂ neutralisiert. Da bei einer Säurezahlbestimmung das bei der Bestimmung benötigte Wasser die sofortige Hydrolyse des Produktes bewirkt und durch diese Reaktion immer mehr Säure freigesetzt wird, war eine Titrationsbestimmung des Säuregehaltes nicht möglich.
Die Säurezahl mußte deshalb theoretisch berechnet werden.
Zur Neutralisation wurde die errechnete Menge an Ca(OH)₂ in das warme Reaktionsprodukt eingetragen und das Gemisch unter Rühren bei 105°C, Durchleitung von 40 l Luft/h und 500 mbar für 30 Minuten behandelt.
Das neutralisierte Produkt (bei 100 bis 105°C hochviskos) wird mit Hilfe einer Druckfilternutsche und einem Loeffler-Filter (80 NMO12) bei 100 bis 105°C unter 3 bar filtriert.
Anschließend wurde das noch heiße Produkt unter ansonsten gleichen Bedingungen über ein Rundfilter (NNG 15 mittelschnell filtrierend) nochmals filtriert.

### Beispiel 2

### Herstellung der Mischungen aus resorbierbaren Wachsen und Reaktivvernetzern und deren Abreaktion

Der Reaktiv-Vernetzer (Monomeres gemäß Beispiel 1,2) wird alternativ mit einem der beiden Wachse nach Beispiel 1.1 a auf Basis Glycolsäure beziehungsweise 1.1 b auf Basis Milchsäure gemischt. Die Eigenschaften der Gemische sind in den Tabellen 3 bis 6 aufgelistet. Zur Herstellung der Polymerisate waren die Mischungen 5 h einer UV-Lichtquelle (Dr. Hönle GmbH; UV ASpot 400/T; 400 W 200/230 V) ausgesetzt.

Im einzelnen gilt dabei zu den Angaben dieser nachfolgenden Tabellen: Die Kürzel "GS" beziehungsweise "EG" bedeuten jeweils Glykolsäure beziehungsweise Ethylenglykol. Die Abreaktion der Stoffgemische aus den Tabellen 3 und 4 erfolgte im UV-Licht ohne Zusatz von Sensibilisator. Die Abreaktion der Stoffgemische aus den Tabellen 5 und 6 erfolgte demgegenüber unter Zusatz eines UV-Sensibilisators - siehe hierzu die Angabe im Kopf der Tabelle 5 -.

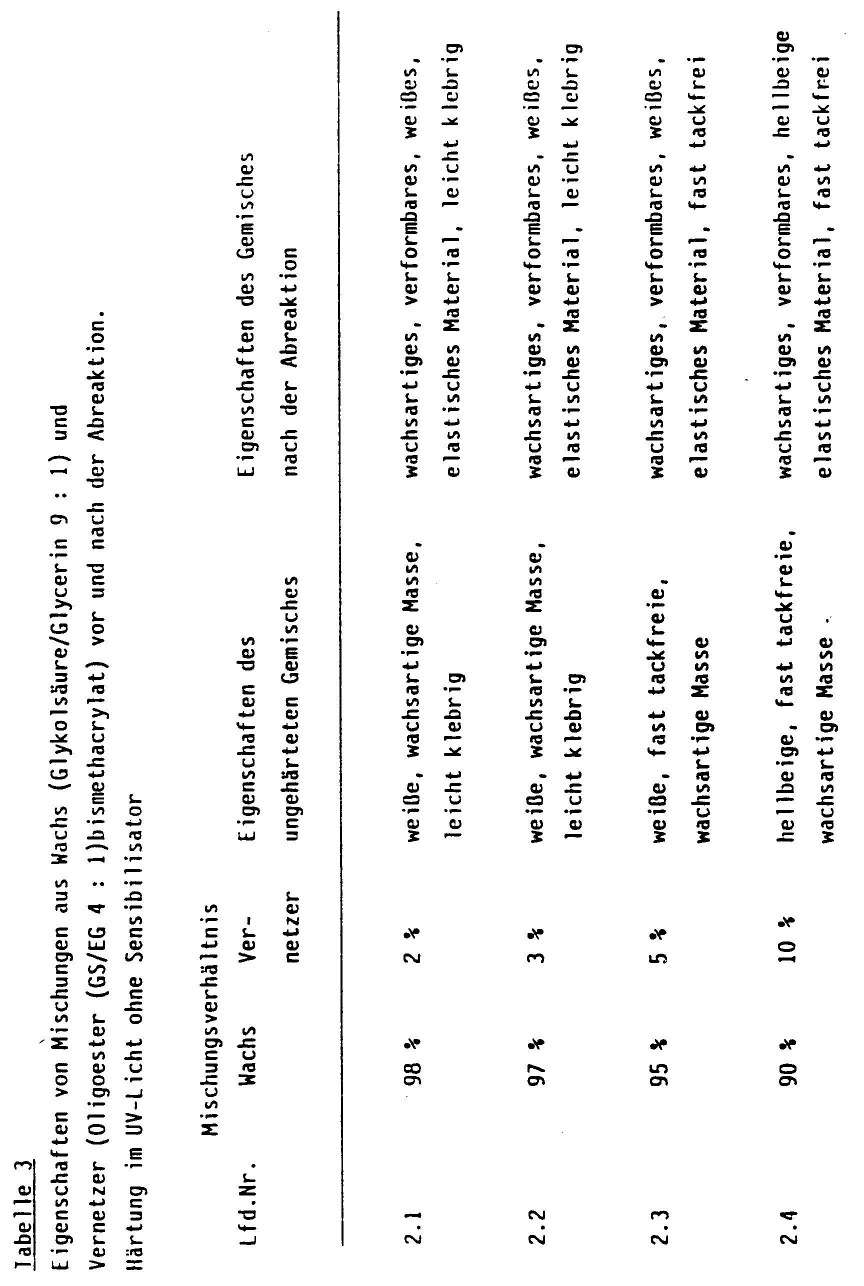

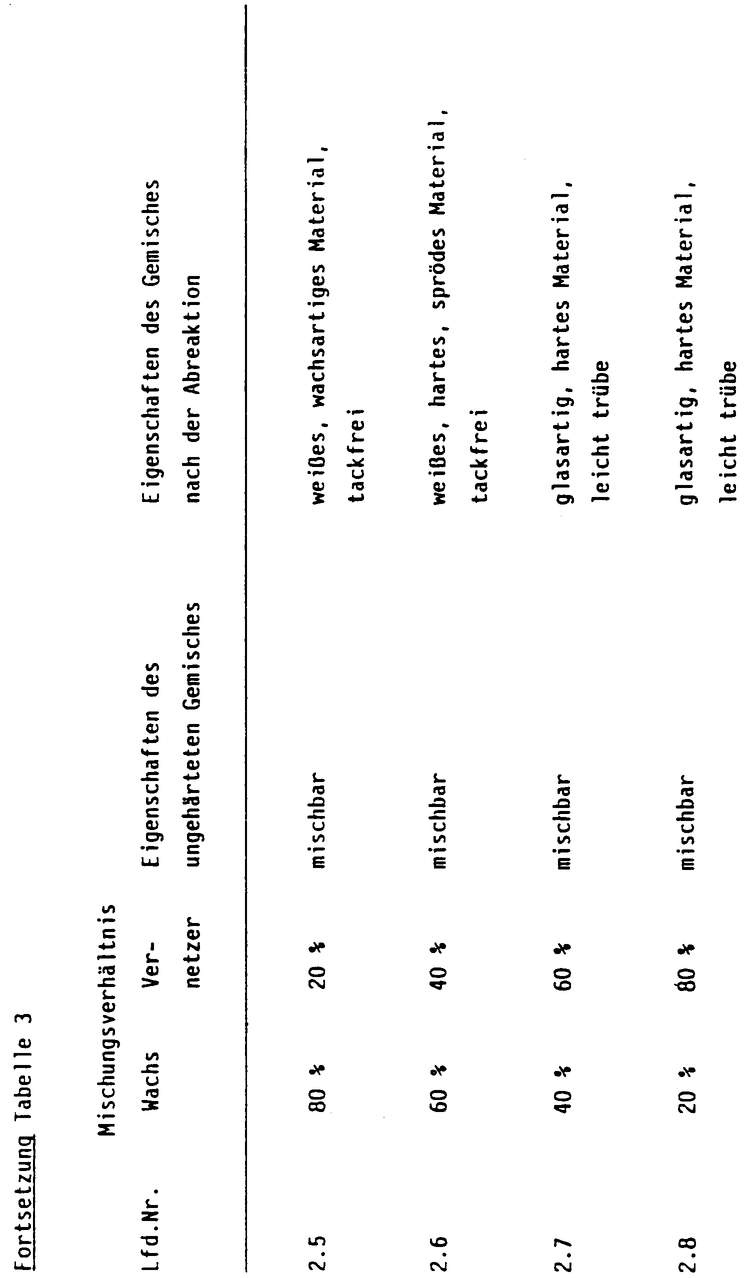

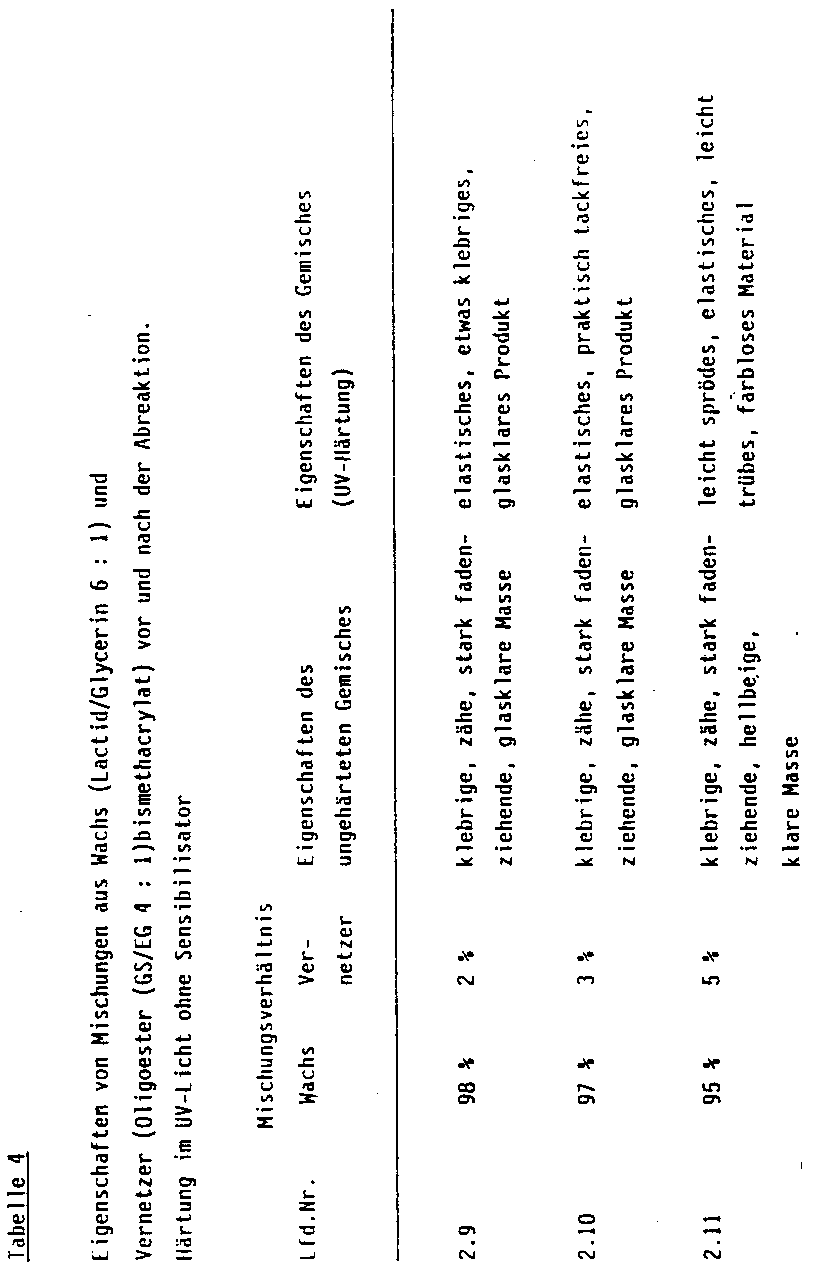

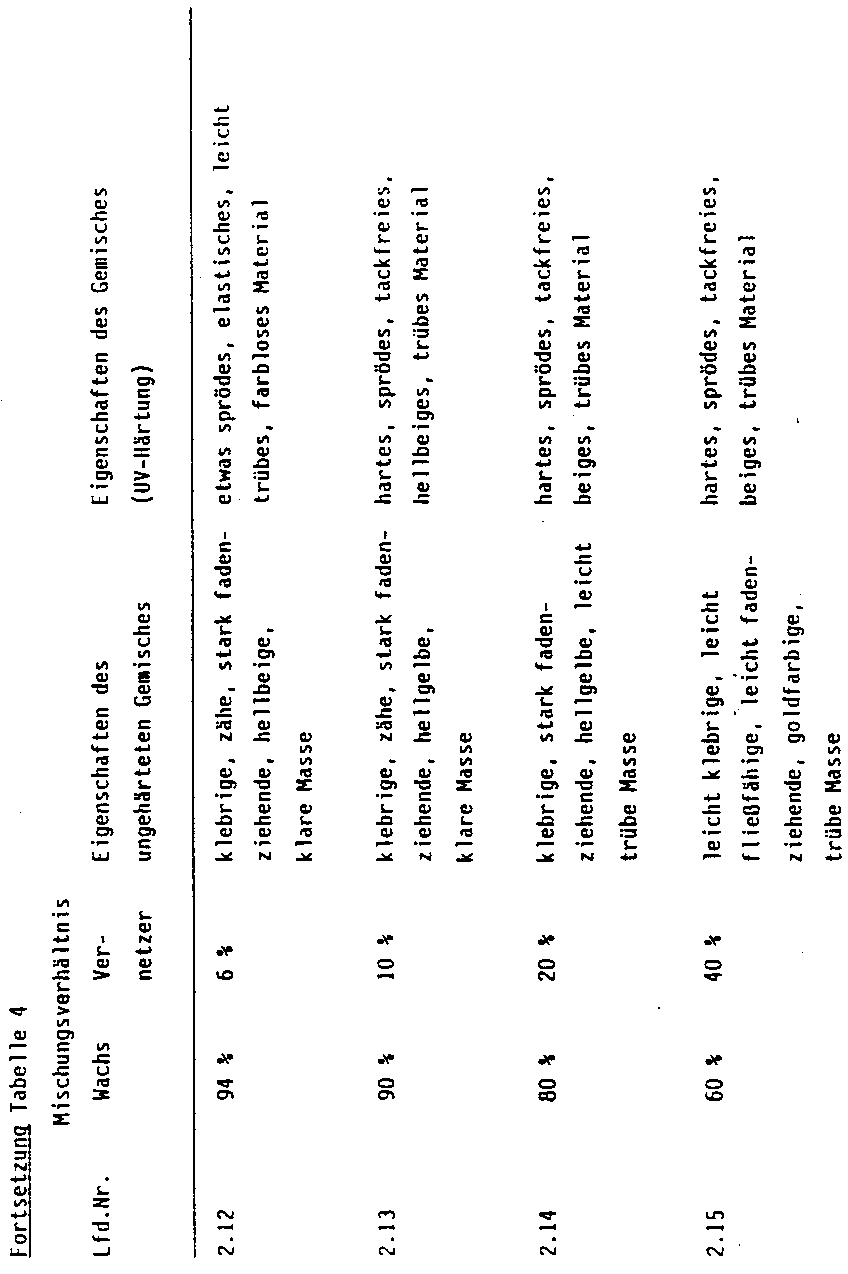

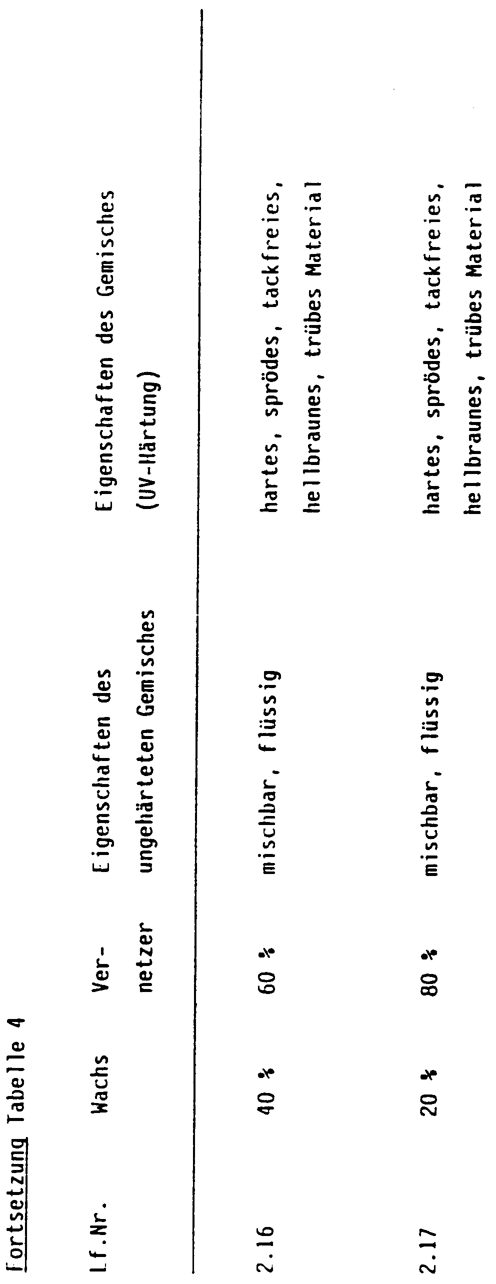

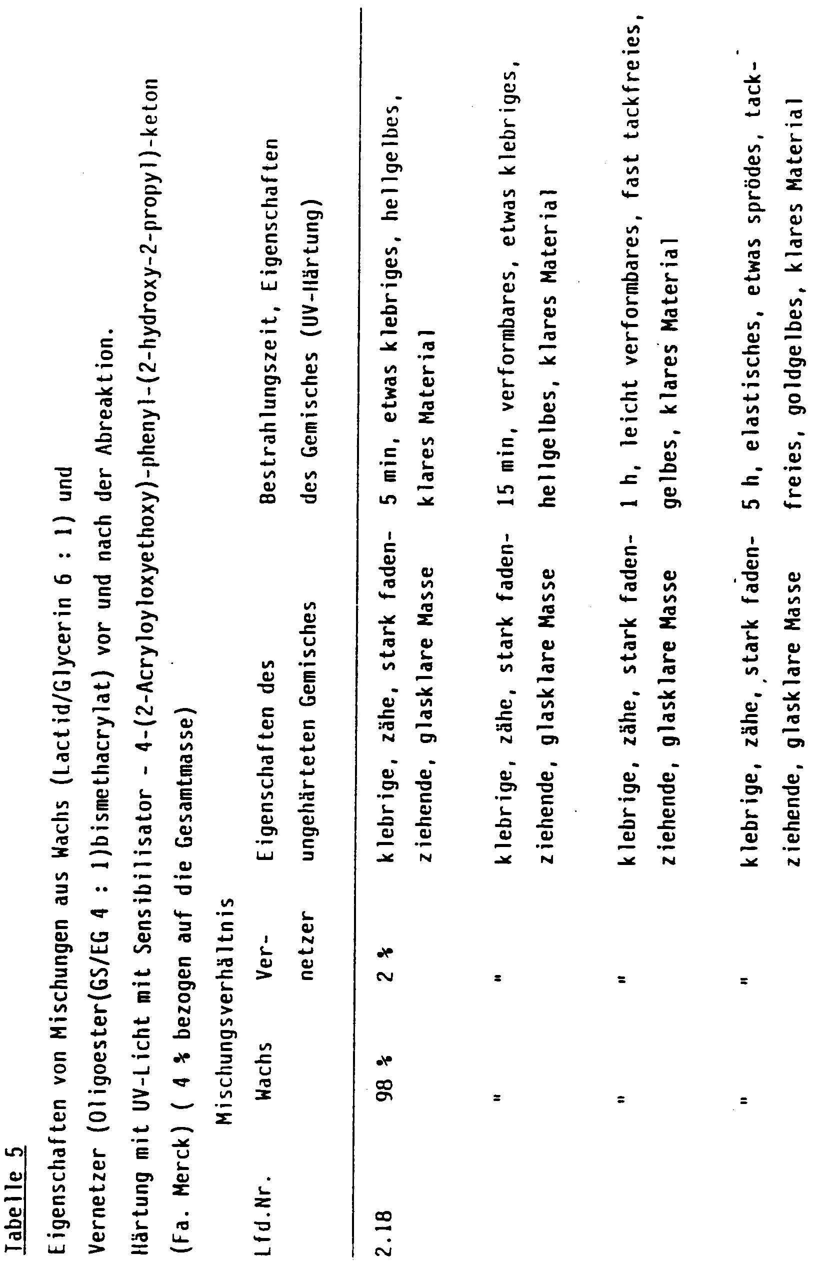

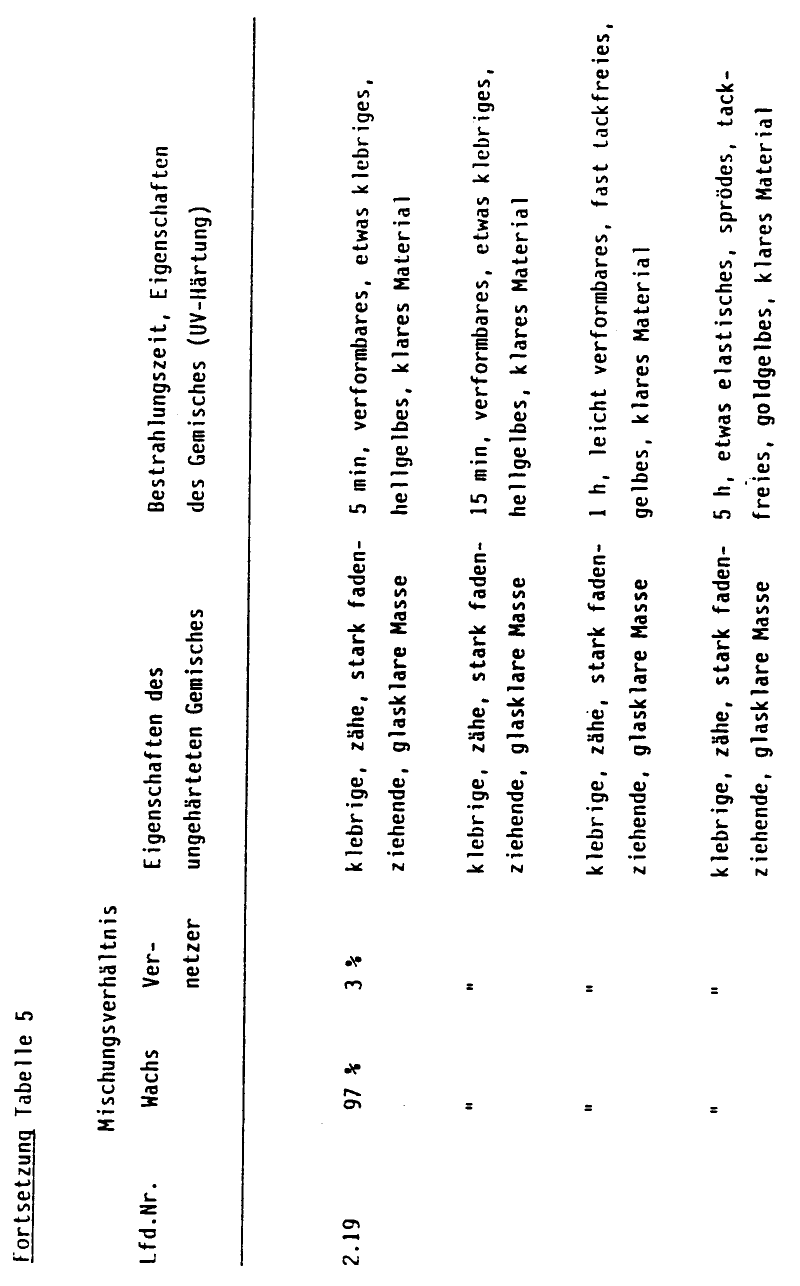

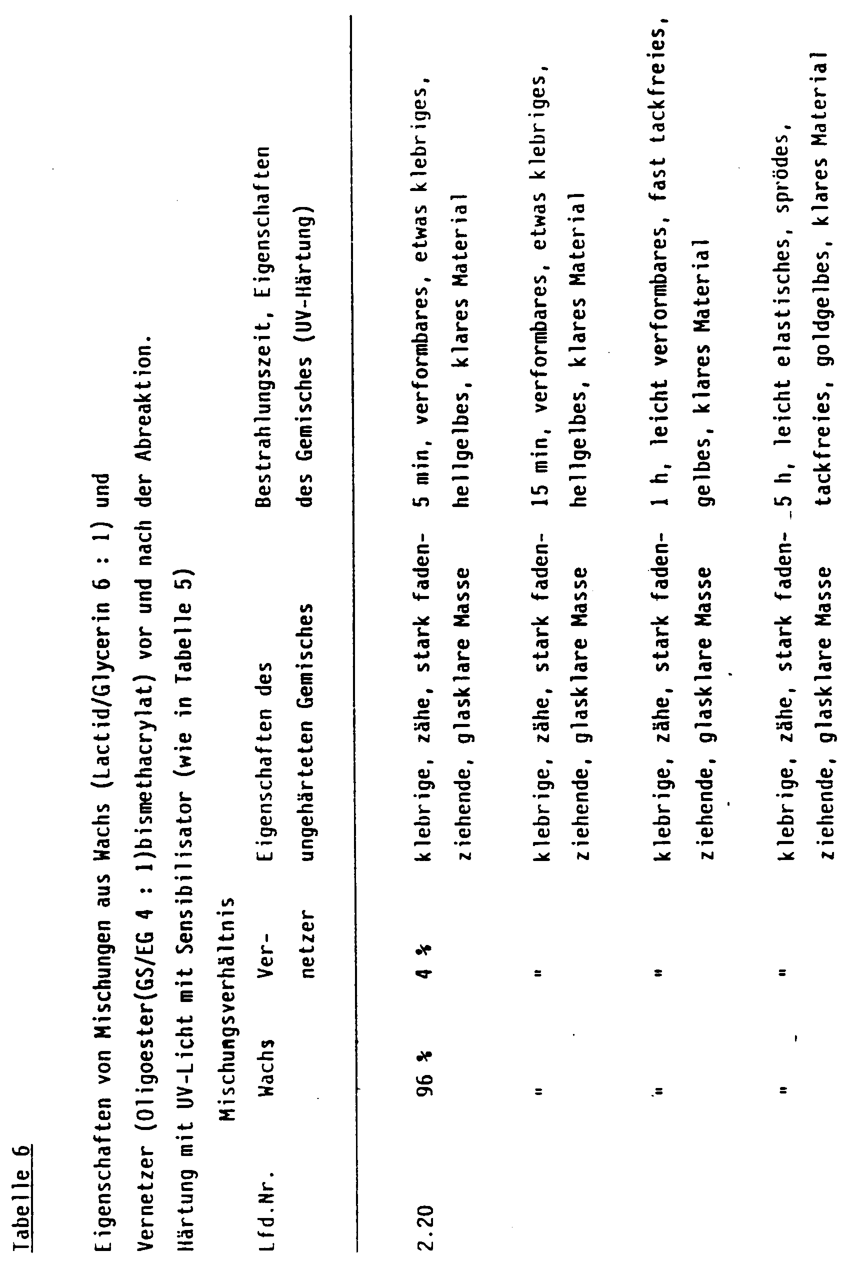

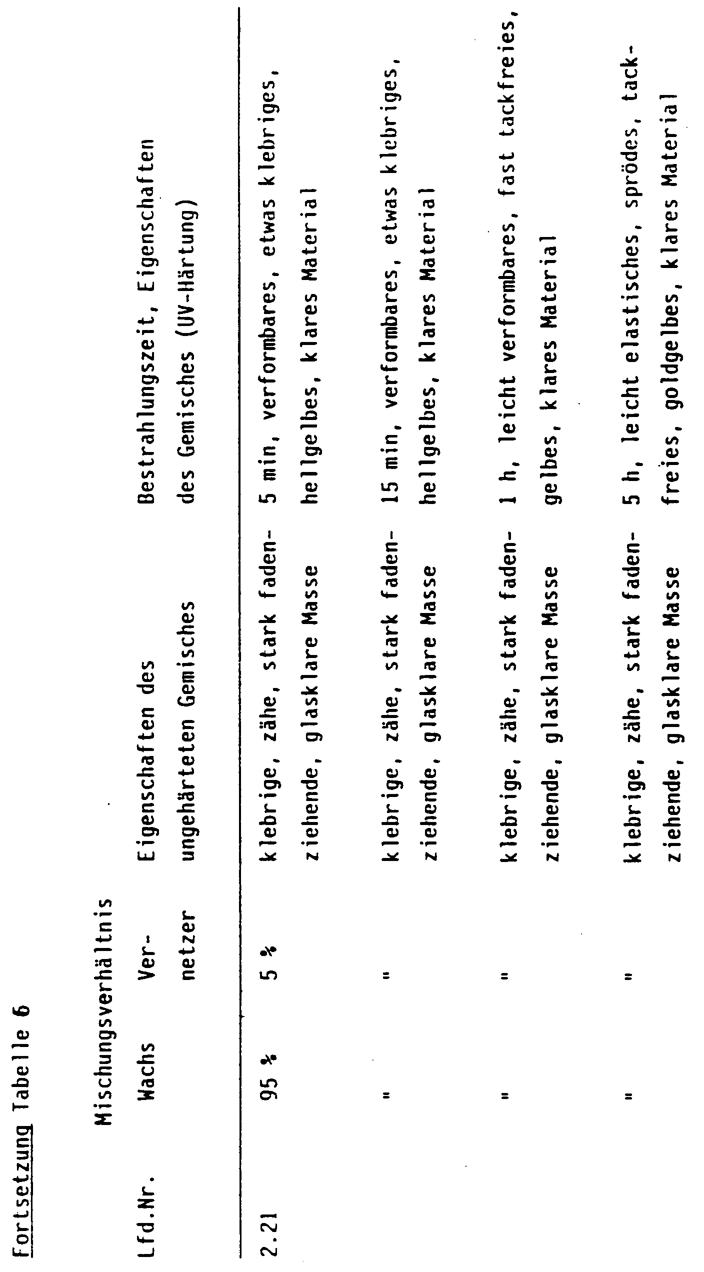

### Beispiel 3

Es werden Wertstoff-beladene Proben eines erfindungsgemäßen Depot-Materials gemäß den nachfolgenden Angaben hergestellt, an denen dann die Wertstoff-Freigabe in einem Versuchsmodell durch Reihenversuche bestimmt wird.

Als Wertstoffe werden verschiedene handelsübliche Antibiotika eingesetzt, die in jeweils vorgegebenen Konzentrationen zunächst homogen in den Träger eingearbeitet werden. Nachfolgend wird der Vernetzer eingemischt und das Mehrkomponentengemisch als etwa 1 bis 2 mm starke, auf eine Teflonfolie aufgetragene Materialschicht der mehrstündigen Einwirkung von UV-Licht ausgesetzt.

Unter den eingesetzten Arbeitsbedingungen (kein Zusatz von Sensibilisator, gekühlte UV-Lampe 500 W, Bestrahlungsdauer 10 h, Temperatur der Mehrkomponentenmasse 28 °C) fällt ein Material mit einer noch nicht tackfreien Oberfläche an. Soweit gewünscht wird mit der nicht gekühlten UV-Lampe zur Klebfreiheit nachgehärtet (15 h, T 80°C).

Im einzelnen gelten die nachfolgenden Angaben:
- Trägermaterial:: Kondensationsprodukt aus Lactid/Glycerin im Molverhältnis 6/1
- Vernetzer:: Oligo(Glykolsäure/Ethylenglykol 4:1)-bis-methacrylat
- Wertstoffe:: Die unter den nachfolgenden Handelsnamen erhältlichen antibiotischen Wirkstoffe Gentamycinsulfat ^{R}, Chloramphenicol^{R} und Tetrazyklin^{R}
- Mischungsverhältnisse Wertstoff/Träger:: 5 Gew. -% und 0,1 Gew.-%
- Vernetzermenge bezogen auf Träger/Wertstoff-Gemisch;: 3 Gew.-%
Die Herstellung der Wertstoff-enthaltenden Depot-Materialien wird wie folgt vorgenommen: Das Trägermaterial auf Oligomilchsäurebasis wird mit dem feinteiligen Antibiotikum in der jeweils vorgegebenen Menge, gegebenenfalls unter leichtem Erwärmen des Trägermaterials, innig vermischt. Bei einer Temperatur von 50°C wird die Vernetzerkomponente homogen eingemischt. Die Masse wird in einer Schichtstärke von 1 bis 2 mm auf eine Teflonfolie gestrichen und der UV-Härtung unterworfen.

### Bestimmung der Retard-Wirkung

Mit Nähr-Agar beschichtete Glasplatten werden mit den jeweils eingesetzten Testbakterien breitflächig und gleichmäßig beimpft. Die zu untersuchenden Materialproben werden mit dem Nährboden in Kontakt gebracht. Nach Bebrütung bei Raumtemperatur über 2 Tage werden die Hemmhöfe gemessen, die sich um die jeweils zu untersuchende Materialprobe gebildet haben. Bestimmt wird dabei der Abstand zwischen der Antibiotika-enthaltenden Probesubstanz und dem äußeren Rand des Hemmhofes. Die Antibiotika-enthaltenden Materialproben werden dann einer stufenweisen Auslaugung in strömenden Leitungswasser unterworfen. Jeweils nach Abschluß einer Auswaschstufe werden in der vorher angegebenen Weise die jetzt noch sich ausbildenden Hemmhöfe auf einer frischen beimpften Nähr-Agar-Platte bestimmt. Dieser Vorgang wird in einer Reihe von Versuchen so lange wiederholt, bis keine Hemmzonen mehr um die untersuchten Materialproben zu erkennen waren.

Als Testbakterien wurden verwendet Staphylococcus aureus (ATCC 6538) und Escherichia coli (ATCC 11229).

Die Wässerung wird in der folgenden Weise vorgenommen: Das zu wässernde Gut wird zwischen zwei Kunststoffschwämme gelegt. Anschließend wird mit einer Handbrause ein gleichmäßiger Wasserstrom durch den Schwamm hindurch ausgebildet. Soweit erforderlich werden die untersuchten Materialproben in ein durchlässiges dünnes Gewebe eingehüllt um Materialverluste durch eventuelles Zerbrechen beim Vorgang des Auswaschens auszuschließen.

Zu einer besseren Beurteilung der Retard-Wirkung erfindungsgemäßer Depot-Materialien mit verzögerter Wirkstofffreigabe wird in einer Paralleluntersuchung unter identischen Arbeitsbedingungen das Auswaschverhalten eines in der operativen Klinikpraxis eingesetzten handelsüblichen Retard-Antibiotika-Präparates untersucht. Das hier eingesetzte Material wird von der Firma Merck, Darmstadt, unter dem Handelsnamen "Septopal^{R}"-10er-Minikette vertrieben. Es handelt sich bei diesem Material des Standes der Technik um ein mit 5 Gew.-% Gentamycinsulfat versetztes Methylmethacrylat-Methylacrylat-Copolymer. Dieser polymere Träger ist nicht körperresorbierbar. Bei der operativen Implantation ist entweder nach einigen Tagen - üblicherweise nach 5 bis 7 Tagen - oder auch nach einem Zeitraum von ca. 1 bis 3 Monaten die komplette Entfernung gegebenenfalls auf operativem Wege erforderlich.

### 3.1

### Eingesetzter Testkeim: Staphylococcus aureus

In einer 6-stufigen Testreihe wird ein 5 Gew.-% Gentamycinsulfat-enthaltendes Retard-Material der eingangs dieses Beispiels 3 angegebenen Zusammensetzung der Wässerung für insgesamt 19 Tage unterworfen. Zu Versuchsbeginn und zwischen den einzelnen Wässerungsstufen werden in der zuvor angegebenen Weise die sich ausbildenden Hemmhöfe um die jeweils untersuchte Materialprobe bestimmt.

Parallel und unter völlig gleichen Bedingungen wird das Langzeitverhalten eines "Septopal^{R}"-Element mitgetestet.

Die Wässerung erfolgt in den folgenden aneinander anschließenden und durch die Bestimmung der Hemmhöfe unterbrochenen Arbeitsstufen:

| | |
|---|---|
| 1. Stufe: | 4 Tage |
| 2. Stufe: | 4 Tage |
| 3. Stufe: | 3 Tage |
| 4. Stufe: | 2 Tage |
| 5. Stufe: | 3 Tage |
| 6. Stufe: | 3 Tage |

Die Größe der jeweils bestimmten Hemmhöfe (in mm) ist in der nachfolgenden Tabelle 7 zusammengefaßt:

**Tabelle 7**

| | Depotmaterial gemäß Beispiel 3 | "Septopal^{R}" |
|---|---|---|
| Hemmhof (mm) | 12 - 15 | 12 - 15 |
| 1. Stufe | 8 | 8 - 10 |
| 2. Stufe | 5 - 7 | 5 - 10 |
| 3. Stufe | 6 - 8 | 7 - 9 |
| 4. Stufe | 5 | 7 |
| 5. Stufe | 6 - 8 | 6 - 8 |
| 6. Stufe | 4 | 2 - 3 |

In einer vergleichbaren Untersuchung wird das erfindungsgemäße Depot-Material der zuvor angegebenen Zusammensetzung mit Gentamycinsulfat in einer Konzentration von 1 Gew.-% versetzt und entsprechend stufenweise gewässert. Die beim Frischmaterial und nach den jeweiligen Stufen bestimmten Hemmhöfe sind die folgenden:

| | |
|---|---|
| Frischmaterial: | 7 - 10 mm |
| 1. Stufe (4 Tage Wässerung) | 5 mm |
| 2. Stufe (4 Tage Wässerung) | 1 - 2 mm |
| 3. Stufe (3 Tage Wässerung) | 3 - 5 mm |
| 4. Stufe (2 Tage Wässerung) | 0 mm |

### 3.2

Vergleichbare Untersuchungen werden mit erfindungsgemäßen Depot-Materialien durchgeführt, die in einer Wertstoffkonzentration von jeweils 0,1 Gew.-% Antibiotikum, die nachfolgenden Wertstoffe enthielten: Gentamycinsulfat, Erythromycin, Tetrazyklin und Chloramphenicol.

Die Wässerungsstufen werden im Rhythmus jeweils eines Tages durchgeführt, dazwischen wird die Größe der noch vorhandenen Hemmhöfe (mm) bestimmt.

Der Blindwert für den Hemmhof (Testkeim: Staph.aureus) liegt für alle eingesetzten Materialproben bei etwa 5 - 6 mm. Das Gentamycinsulfatenthaltende Depot-Material bleibt in seiner Wirksamkeit bis zum 3. Wässerungstag praktisch unverändert, von da an tritt ein nahezu linearer Abfall der Hemmhofgröße bis zum Wert 0 am Ende des 7. Wässerungstages ein. Etwa vergleichbar ist der Verlauf der Hemmhofkurve bei dem Erythromycin-enthaltenden Retard-Material. Hier ist allerdings erst nach Ablauf des 8. Wässerungstages der Wert 0 erreicht. Im Zeitraum der Wässerungsstufen 5 bis 7 liegen im Vergleich zum Gentamycinsulfat-enthaltenden Präparat verbesserte Hemmhofwerte vor.

Das Tetrazyklin-enthaltende Retard-Präparat zeigt nach der ersten Wässerungsstufe kaum einen Wirkungsabfall, dann allerdings tritt rascher Wirkungsverlust ein. Das Chloramphenicol-enthaltende Präparat läßt bereits am Ende des 1. Wässerungstages einen Wirkungsverlust auf weniger als 50 % des Einsatzwertes erkennen.

Wird jetzt allerdings die Wirkstoffkonzentration im Retard-Präparat auf 5 Gew.-% erhöht und als Trägermaterial ein Oligomeres auf Basis Glycerin/Glykolsäure/Milchsäure im Molverhältnis 1/2/10 eingesetzt, dann sinkt der Blindwert des Hemmhofes beim Tetrazyklin-enthaltenden Präparat (42 mm) innerhalb eines Wässerungszeitraumes von 8 Tagen nur vergleichsweise unbedeutend um etwa 10 Einheiten ab (31 mm). Etwas stärker ist der Aktivitätsverlust innerhalb dieses Zeitraumes der Materialproben mit einerseits Gentamycinsulfat und andererseits mit Erythromycin als Antibiotikum. Auch hier bleibt aber jeweils eine antibiotische Aktivität von mehr als 50 % des Blindwertes erhalten.

Entsprechende Untersuchungen an Nährboden mit E.coli als Testkeime bestätigen die Retard-Wirkung in der Freigabe der antibiotischen Substanzen im Sinne der erfindungsgemäßen Lehre.

## Patentansprüche

1. Medizinische und/oder biologische Wertstoffe-enthaltende Retard-Systeme auf Basis von Oligomer- und/oder Polymerverbindungen niederer Hydrocarbonsäuren mit 2 - 6 C-Atomen als Trägermaterial mit verzögerter Wirkstoffreigabe, dadurch gekennzeichnet, daß sie mit Polymerverbindungen aus olefinisch ungesättigten Estern niederer Hydroxycarbonsäuren mit 2 bis 6 C-Atomen und/oder deren Oligomeren (Vernetzerkomponenten) modifiziert sind.

2. Retard-Systeme nach Anspruch 1, dadurch gekennzeichnet, daß sie als Modifizierungsmittel wenigstens anteilsweise Polymere olefinisch mehrfunktioneller Vernetzerkomponenten der angegebenen Art enthalten, wobei 2- bis 4-funktionelle olefinisch reaktive Hydroxycarbonsäurederivate bevorzugt sein können.

3. Retard-Systeme nach Ansprüchen 1 und 2, dadurch gekennzeichnet, daß sie als Trägermaterial mit zeitverzögerter Wirkstofffreigabe Oligomer- und/oder Polymerverbindungen von Hydroxycarbonsäuren mit 2 bis 4 C-Atomen und insbesondere solche Verbindungen der Milchsäure und/oder der Glykolsäure enthalten, wobei weiterhin auch die bevorzugten Vernetzerkomponenten reaktive Derivate von Hydroxycarbonsäuren der angegebenen C-Zahlbereiche sind und sich insbesondere von der Milchsäure und/oder der Glykolsäure ableiten.

4. Retard-Systeme nach Ansprüchen 1 bis 3, dadurch gekennzeichnet, daß sie zum Einsatz im und/oder am menschlichen und/oder tierischen Organismus geeignet und dabei insbesondere zur Implantation in den lebenden Körper und/oder zur topischen Verabreichung ausgebildet sind.

5. Retard-Systeme nach Ansprüchen 1 bis 4, dadurch gekennzeichnet, daß die Vernetzerkomponenten wenigstens anteilig Ester und/oder Oligoester niederer mehrfunktioneller Alkohole oder Carbonsäuren mit Milchsäure und/oder Glykolsäure und endständigen olefinisch ungesättigten Resten sind, wobei sich bevorzugte Vernetzerkomponenten von niederen 2- bis 4-wertigen Alkoholen, insbesondere von Ethylenglykol und/oder Glycerin ableiten und mittels (Meth)acrylsäure-Resten olefinisch substituiert sind.

6. Retard-Systeme nach Ansprüchen 1 bis 5, dadurch gekennzeichnet, daß ihre Vernetzerkomponenten Umsetzungsprodukte von Glycerinestern der Milchsäure und/oder ihren Oligomeren und (Meth)acrylsäure und/oder deren reaktionsfähigen Derivaten sind, die bevorzugt im statistischen Mittel 2 bis 3 olefinische Endgruppen/Mol Glycerin aufweisen.

7. Retard-Systeme nach Ansprüchen 1 bis 6, dadurch gekennzeichnet, daß sie als Trägermaterial mit zeitverzögerter Wirkstofffreigabe Oligernere und/oder Polymere der niederen Hydroxycarbonsäuren mit zäh-viskoser bis fester Beschaffenheit bei Raumtemperatur aufweisen, wobei für den Einsatzbereich der Implantation in den lebenden Körper als Oligomerverbindungen auch hier bevorzugt Ester niederer mehrfunktioneller Alkohole, insbesondere des Ethylenglykols und/oder des Glycerins, vorliegen, während für den Einsatzbereich der topischen Anwendung im Trägermaterial wenigstens anteilsweise auch Oligomerverbindungen mit endständigen Carboxylgruppen eingesetzt werden können.

8. Retard-Systeme nach Ansprüchen 1 bis 7, dadurch gekennzeichnet, daß als Trägermaterial mit Zeitverzögerter Wirkstofffreigabe wenigstens anteilsweise Oligoester mit mittleren Molekulargewichten im Bereich von etwa 200 bis 5.000, bevorzugt etwa 300 bis 2.000, vorliegen, wobei Glycerin/Oligo-Milchsäureester mit mittleren Molgewichten im Bereich von etwa 200 bis 1.500, insbesondere im Bereich von etwa 300 bis 1.000 besonders bevorzugt sein können.

9. Retard-Systeme nach Ansprüchen 1 bis 8, dadurch gekennzeichnet, daß sie die modifizierenden und wenigstens anteilsweise abreagierten Vernetzerkomponenten in untergeordneten Mengen - bezogen auf Gewicht des Trägers mit verzögerter Wirkstofffreigabe - enthalten, wobei Mischungsverhältnisse von etwa 0,5 bis 40 Gew.-%, vorzugsweise etwa 1 bis 20 Gew.-% Vernetzer - Gew.-% hier bezogen auf die Summe von Träger und Vernetzer - bevorzugt sein können und insbesondere der Gehalt an Vernetzerkomponenten unterhalb etwa 10 Gew.-% liegt.

10. Retard-Systeme nach Ansprüchen 1 bis 9, dadurch gekennzeichnet, daß das Trägermaterial und die polymeren Vernetzerkomponenten zusammen mit den Wertstoffen entweder in weitgehend homogener Abmischung miteinander vorliegen und/oder derart in zum Beispiel schichtförmiger Anordnung im Retard-System vorliegen, daß wenigstens weitgehend Vernetzer-freie Bereiche des Wertstoff/Trägergemisches von Materialbereichen mit einem Gehalt an abreagierten Vernetzerkomponenten umhüllt sind.

11. Retard-Systeme nach Ansprüchen 1 bis 10, dadurch gekennzeichnet, daß das Trägermaterial eine wenigstens bimodale Strukturbeschaffenheit aufweist und dabei Oligo- und/oder Polyester vergleichsweise hoher Zerfallsgeschwindigkeit unter Einsatzbedingungen, insbesondere im lebenden Körper, mit Oligo- und/oder Polyestern langsamerer Abbaugeschwindigkeit vereinigt, wobei sich die Materialanteile hoher Abbaugeschwindigkeit bevorzugt von Glykolsäure und die langsamer abbauenden Materialanteile sich bevorzugt von der Milchsäure ableiten.

12. Retard-Systeme nach Ansprüchen 1 bis 11, dadurch gekennzeichnet, daß sie als Trägermaterial wenigstens weitgehend homogene Abmischungen von Oligomeren beziehungsweise Polymeren auf Milchsäurebasis und solchen auf Glykolsäurebasis enthalten, wobei aber auch mehrphasige Abmischungen diskreter Materialbereiche der Einzelkomponenten des Trägermaterials vorliegen können.

13. Retard-Systeme nach Ansprüchen 1 bis 12, dadurch gekennzeichnet, daß - insbesondere im Fall mehrphasiger Gemische des Trägermaterials - auch die polymeren Vernetzerkomponenten nach Anordnung, Art und/oder Menge mehrphasig im Retard-System vorliegen können.

14. Retard-Systeme nach Ansprüchen 1 bis 13, dadurch gekennzeichnet, daß sie als Wertstoffe medikamentös wirksame Komponenten, insbesondere Antibiotika, Zytostatika, Cortison und/oder Hormone enthalten und dabei insbesondere für die Implantation in lebendes Gewebe ausgebildet sind oder aber Wirkstoffe aus den Bereichen des Pflanzenschutzes beziehungsweise der Förderung des Pflanzenwachstums als Wertstoff enthalten.

15. Verwendung von radikalisch reaktiven Vernetzerkomponenten auf Basis olefinisch ungesättigter Ester niederer Hydroxycarbonsäuren mit 2 bis 6 C-Atomen und/oder deren Oligomeren zur Modifizierung von Depot-Materialien, insbesondere Retard-Systemen, die medizinische und/oder biologische Wertstoffe in Abmischung mit einem Trägermaterial bei zeitverzögerter Wirkstoffabgabe im praktischen Einsatz enthalten.

16. Ausführungsform nach Anspruch 15, dadurch gekennzeichnet, daß die Vernetzer in der Applikationsform des Retard-Systems als Polymerverbindinngen vorliegen und dabei in weitgehend homogener oder auch ungleichmäßiger Verteilung im zu applizierenden Retard-System angeordnet sind.

17. Ausführungsform nach Ansprüchen 15 und 16, dadurch gekennzeichnet, daß die modifizierenden Polymerverbindungen aus den radikalisch reaktiven Vernetzerkomponenten in Abmischung mit wenigstens einem Anteil des Depot-Materials in-situ hergestellt worden sind.

18. Ausführungsform nach Ansprüchen 15 bis 17, dadurch gekennzeichnet, daß mehrfunktionelle Vernetzerkomponenten auf Basis von Milchsäure- und/oder Glykolsäure-Estern eingesetzt werden, die wenigstens anteilig 2 bis 4, vorzugsweise 2 und/oder 3 olefinische Gruppen im Molekül aufweisen.

19. Ausführungsform nach Ansprüchen 15 bis 18, dadurch gekennzeichnet, daß als Vernetzerkomponenten mehrfunktionelle (Meth)acrylsäure-Ester von Hydroxyl-terminierten Reaktionsprodukten aus der Umsetzung von niederen mehrfunktionellen Alkoholen, insbesondere Ethylenglykol und/oder Glycerin, mit Milchsäure und/oder Glykolsäure verwendet werden.

20. Ausführungsform nach Ansprüchen 15 bis 19, dadurch gekennzeichnet, daß die Vernetzerkomponenten entweder in das Träger/Wertstoff-Gemisch eingemischt und dort zur Abreaktion gebracht worden sind und/oder daß ein Träger/Wertstoff-Gemisch mit einer abreagierte Härterkomponenten enthaltenden Materialschicht umhüllt ist.

21. Ausführungsform nach Ansprüchen 15 bis 20, dadurch gekennzeichnet, daß als Trägermaterial körperverträgliche Oligomere und/oder Polymere von niederen Hydroxycarbonsäuren, insbesondere auf Basis Milchsäure und/oder Glykolsäure, vorliegen, die bevorzugt mittlere Molekulargewichte im Bereich von etwa 200 bis 5.000, insbesondere etwa 300 bis 2.000, aufweisen.

## Claims

1. Retard systems which contain medicinal and/or biological valuable materials and have been based on oligomer and/or polymer compounds of lower hydroxycarboxylic acids having from 2 to 6 carbon atoms as carrier materials providing a sustained release of the active substance, characterized in that said retard systems and/or depot materials have been modified with polymer compounds prepared from olefinically unsaturated esters of lower hydroxycarboxylic acids having from 2 to 6 carbon atoms and/or oligomers thereof (cross-linker components).

2. The retard systems according to claim 1, characterized in that they at least portionwise contain polymers of olefinically polyfunctional cross-linker components of the type indicated as modifying agents, wherein di- to tetrafunctional olefinically reactive hydroxycarboxylic acid derivatives may be preferred.

3. The retard system according to claims 1 and 2, characterized in that they contain oligomer and/or polymer compounds of hydroxycarboxylic acids having from 2 to 4 carbon atoms, and especially such compounds of lactic acid and/or glycolic acid as carrier materials providing a sustained release of the active substance, wherein the preferred cross-linker components are reactive derivatives of hydroxycarboxylic acids of the ranges of numbers of carbon atoms as indicated and especially those derived from lactic acid and/or glycolic acid.

4. The retard systems according to claims 1 to 3, characterized in that they are suitable for use in and/or on the human and/or animal organism and, more specifically have been provided for implantation into the living organism and/or for topical administration.

5. The retard systems according to claims 1 to 4, characterized in that the cross-linker components at least portionwise are esters and/or oligoesters of lower polyfunctional alcohols or carboxylic acids with lactic acid and/or glycolic acid and have terminal olefinically unsaturated groups, wherein preferred cross-linker components are derived from lower di- to tetrahydric alcohols, and more specifically from ethyleneglycol and/or glycerol, and have been olefinically substituted with (meth)acrylic acid moieties.

6. The retard systems according to claims 1 to 5, characterized in that the cross-linker components thereof are reaction products of glycerol esters of lactic acid and/or its oligomers and (meth)acrylic acid and/or reactive derivatives thereof which, as a random average, comprise from 2 to 3 olefinic terminal groups per one mole of glycerol.

7. The retard systems according to claims 1 to 6, characterized in that they contain oligomers and/or polymers of the lower hydroxycarboxylic acids having a tough-viscous to solid nature at room temperature as carrier materials providing a sustained release of the active substance, wherein for the range of use for implantation into the living organism it is preferred also here that esters of lower functional alcohols, and especially of ethyleneglycol and/or glycerol, are present as the oligomer compounds, whereas for the range of use of a topical application also oligomer compounds having terminal carboxyl groups can be employed at least portionwise in the carrier material.

8. The retard systems according to claims 1 to 7, characterized in that oligoesters having average molecular weights within the range of from about 200 to 5,000, and preferably of from about 300 to 2,000, are present as carrier materials providing a sustained release of the active substance, wherein glycerol/oligo-lactic acid esters having average molecular weights within the range of from about 200 to 1,500, and more specifically within the range of from about 300 to 1,000, can be especially preferred.

9. The retard systems according to claims 1 to 8, characterized in that they contain the modifying and at least partially reacted cross-linker components in minor amounts - relative to the weight of the carrier materials providing a sustained release of the active substance - , wherein mixing ratios of from about 0.5 to 40% by weight, and preferably of from about 1 to 20% by weight of cross-linker - % by weight here relative to the sum of carrier material and cross-linker components - may be preferred and, more particularly, the content of cross-linker components is less than about 10% by weight.

10. The retard systems according to claims 1 to 9, characterized in that the carrier material and the polymeric cross-linker components are present in the retard system in combination with the valuable materials either in a largely homogeneous admixture and/or, for example, in a layered arrangement such that regions which are at least largely free from cross-linker of the valuables/carrier mixtures are enclosed by material regions having a content of reacted cross-linker components.

11. The retard systems according to claims 1 to 10, characterized in that the carrier material has an at least bimodal structural nature and thereby combines oligo- and/or polyesters having a comparably high decay rate under the conditions of use, especially in the living organism, with oligo- and/or polyesters having a slower decay rate, wherein the material portions of high decay rate are preferably derived from glycolic acid, and the material portions of slower decay rate are preferably derived from lactic acid.

12. The retard systems according to claims 1 to 11, characterized in that they contain at least largely homogeneous admixtures of oligomers and/or polymers derived from lactic acid and those derived from glycolic acid as the carrier material, wherein also multiphase admixtures of discrete material regions of the individual components of the carrier material may be present.

13. The retard systems according to claims 1 to 12, characterized in that - especially in the case of multiphase mixtures of the carrier material- also the polymeric cross-linker components may be present in the retard system in the multiphase state according to configuration, kind and/or amount.

14. The retard systems according to claims 1 to 13, characterized in that they contain components having medicament activity as valuable materials, and especially antibiotics, cytostatics, cortisone and/or hormones and thereby, more particularly, have been designed for implantation into the living organism, or contain active substances from the area of crop protection and/or plant growth promotion as valuable material.

15. Use of cross-linker components which are reactive via free radicals, said components having been based on olefinically unsaturated esters of lower hydroxycarboxylic acids having from 2 to 6 carbon atoms and/or oligomers thereof, for modifying depot materials, and especially retard systems, which contain medicinal and/or biological valuable materials in admixture with a carrier material in a sustained release of the active substance(s) in practical application.

16. The embodiment according to claim 15, characterized in that in the application form of the retard system the cross-linkers are present as polymer compounds and therein have been arranged in a largely homogeneous or also non-uniform distribution in the retard system to be applied.

17. The embodiment according to claims 15 and 16, characterized in that the modifying polymer compounds have been prepared *in situ* from the free radical-reactive cross-linker components in admixture with at least a proportion of the depot material.

18. The embodiment according to claims 15 to 17, characterized in that polyfunctional cross-linker components are employed which have been derived from lactic acid and/or glycolic acid esters, said components containing at least portionwise from 2 to 4, and preferably at least 2 and/or 3, olefinic groups in the molecule.

19. The embodiment according to claims 15 to 18, characterized in that polyfunctional (meth)acrylic acid esters of hydroxy-terminated reaction products from the reaction of lower polyfunctional alcohols, and especially ethyleneglycol and/or glycerol, with lactic acid and/or acid or glycolic acid are used as the cross-linker components.

20. The embodiment according to claims 15 to 19, characterized in that either the cross-linker components are admixed into the carrier/valuables mixture and are allowed to react therein, and/or that a carrier/valuables mixture is enclosed by a material layer containing reacted curing components.

21. The embodiment according to claims 15 to 20, characterized in that body-compatible oligomers and/or polymers of lower hydroxycarboxylic acids, especially those based on lactic acid and/or glycolic acid, are present as carrier material, which components preferably have average molecular weights within the range of from about 200 to 5,000, and especially of from about 300 to 2,000.

## Revendications

1. Systèmes retard renfermant des substances de valeur médicales et/ou biologiques à base de combinaisons oligomères et/ou polymères d'acide hydroxycarboxylique inférieur ayant de 2 à 6 atomes de carbone, en tant que matériau support ayant une libération retardée de principe actif, caractérisés en ce qu'ils sont modifiés par des combinaisons polymères à base d'esters oléfiniques insaturés d'acide hydroxycarboxylique inférieur ayant de 2 à 6 atomes de carbone et/ou de leurs oligomères (composants de réticulation).

2. Systèmes retard selon la revendication 1, caractérisé en ce qu'ils renferment, comme agent de modification, au moins partiellement, de polymères des composants de réticulation oléfiniques plurifonctionnels du type mentionné, tandis que des combinaisons d'acide 2,4-hydroxycarboxylique réactifs oléfiniquement fonctionnels peuvent être préférés.

3. Systèmes retard selon les revendications 1 et 2, caractérisés en ce qu'ils renferment comme matériau support avec libération retardée dans le temps, de principe actif, les combinaisons oligomères et/ou polymères d'acides hydroxycarboxyliques ayant de 2 à 4 atomes de carbone et en particulier ces combinaisons de l'acide lactique et/ou de l'acide glycolique, tandis que en outre aussi les composants de réticulation préférés sont des combinaisons réactifs d'acides hydroxycarboxyliques de la zone indiquée de nombre de carbone et qui dérivent en particulier de l'acide lactique et/ou de l'acide glycolique.

4. Systèmes retard selon les revendications 1 à 3, caractérisés en ce qu'ils conviennent pour l'utilisation dans et/ou sur l'organisme humain et/ou animal et de cette façon sont formés en particulier pour l'implantation dans le corps vivant et/ou pour l'administration topique.

5. Systèmes retard selon les revendications 1 à 4, caractérisés en ce que les composants de réticulation sont au moins en partie des esters et/ou des oligoesters d'alcools inférieurs plurifonctionnels ou d'acides carboxyliques avec l'acide lactique et/ou l'acide glycoliques et des radicaux non saturés terminaux oléfiniques, parmi lesquels des composants préférés de réticulation dérivent d'alcools inférieurs bi- à quadrivalents, en particulier de l'éthylène glycol et/ou du glycérol et sont substitués oléfiniquement à l'acide de radicaux d'acide (méth)acrylique.

6. Systèmes retard selon les revendications 1 à 5, caractérisés en ce que leurs composants de réticulation sont des produits de réaction d'esters du glycérol et d'acide lactique et/ou de leurs oligomères et d'acide (méth)acrylique et/ou leurs combinaisons aptes à la réaction, qui possèdent, de préférence en moyenne statistique 2 à 3 groupes terminaux oléfiniques/mol de glycérol.

7. Systèmes retard selon les revendications 1 à 6, caractérisés en ce qu'ils possèdent comme matériau support avec libération retardée dans le temps du principe actif, des oligomères et/ou des polymères des acides hydroxycarboxyliques inférieurs ayant une nature visqueuse a solide à température ambiante, tandis que dans le domaine d'utilisation de l'implantation dans les corps vivants, comme les combinaisons d'oligomères aussi ici de préférence, sont présents des esters d'alcool plurifonctionnels inférieurs, en particulier de l'éthylène glycol et/ou du glycérol, alors que pour le domaine d'utilisation de l'utilisation topique, on peut mettre en jeu dans le matériau support au moins partiellement aussi des combinaisons oligomères avec des groupes carboxyle terminaux.

8. Systèmes retard selon les revendications 1 à 7, caractérisés en ce que sont présents comme matériau support avec une libération retardée dans le temps, au moins partiellement des oligoesters ayant un poids moléculaire moyen dans la zone environ de 200 à 5 000, de préférence environ 300 à 2 000, parmi lesquels les esters de glycéryle/oligo d'acide lactique avec un poids moléculaire moyen dans la zone environ de 200 à 1 500, en particulier dans la zone environ de 300 à 1 000 peuvent être particulièrement préféré.

9. Systèmes retard selon les revendications 1 à 8, caractérisés en ce qu'ils renferment les composants de réticulation qui doivent être modifiés et qui ont réagi au moins partiellement en quantités inférieures, rapporté au poids du support avec libération retardée de principe actif - tandis que les rapports de mélange environ de 0,5 à 40 % en poids - de préférence environ de 1 à 20 % en poids d'agent de réticulation - le % en poids rapporté ici à la somme du support et de l'agent de réticulation - peuvent être préférés et en particulier la teneur en composants de réticulation se situe en dessous environ de 10 % en poids.

10. Systèmes retard selon les revendications 1 à 9, caractérisés en se que le matériau support et les composants d'agents de réticulation polymères se présentent conjointement avec les substances de valeur, ou dans un mélange largement homogène l'un avec l'autre et/ou de cette façon en disposition lamellaire par exemple dans le système retard, en ce que les zones dépourvues au moins largement d'agent de réticulation du mélange substance de valeur/support sont recouverts par des zones de matériau avec une teneur en composants d'agent de réticulation qui a réagi.

11. Systèmes retard selon les revendications 1 à 10, caractérisés en se que le matériau support possède une constitution structurelle au moins bimodale et ainsi réunit des oligo et/ou des polyesters ayant une vitesse de dégradation relativement élevée dans les conditions d'utilisation, en particulier dans les corps vivants, avec des oligo et/ou des polyesters de vitesse de décomposition plus lente, dans lequel les quantités de matériaux de vitesse élevée de décomposition dérivent de préférence d'acide glycolique et les quantités de matériaux qui se décomposent plus lentement dérivent de préférence d'acide lactique.

12. Systèmes retard selon les revendications 1 à 11, caractérisés en ce qu'ils renferment somme matériau support au moins des mélanges largement homogènes d'oligomères ou de polymères à base d'acide lactique et renferment ceux à base d'acide glycolique, tandis que cependant aussi des mélanges à plusieurs phases de zones de matériaux discrets des composants individuels du matériau support peuvent être présents.

13. Systèmes retard selon les revendications 1 à 12, caractérisés en ce que - en particulier dans le cas de mélanges à phases multiples de matériau support - aussi les composants d'agent de réticulation polymères peuvent se présenter selon la disposition, l'art et la manière et/ou la quantité d'une manière à phases multiples dans le système retard.

14. Systèmes retard selon les revendications 1 à 13, caractérisés en ce qu'ils renferment comme substances de valeur, des composants actifs comme médicament, en particulier des antibiotiques, des cytostatiques, de la cortisone et/ou des hormones et pour cela, en particulier, ces composants sont formés pour l'implantation dans le tissu vivant, ou renferment cependant des principes actifs provenant des domaines de la protection des plantes ou de stimulation de la croissance des plantes comme substance de valeur.

15. Utilisation de composants d'agent de réticulation réactifs radicalairement, à base d'esters oléfiniquement non saturés d'acides hydroxycarboxyliques inférieurs ayant de 2 à 6 atomes de carbone et/ou leur oligomères pour la modification des matériaux dépôt, en particulier les systèmes retard qui renferment des substances de valeur médicales et/ou biologiques, en mélange avec un matériau support pour la libération de principe actif retardée dans le temps, en utilisation pratique.

16. Mode d'exécution selon la revendication 15, caractérisé en ce que les agents réticulants dans la forme d'application du système retard se présentent en tant que combinaisons polymères et sont disposés pour cela en distribution largement homogène ou aussi irrégulière dans le système retard qui doit être appliqué.

17. Mode d'exécution selon les revendications 15 et 16, caractérisé en ce que les combinaisons polymères se modifiant, ont été préparés « in situ » à partir des composants d'agent de réticulation réactif radicalairement en mélange avec au moins une fraction de matériau dépôt.

18. Mode d'exécution selon les revendications 15 à 17, caractérisé en ce que l'on met en oeuvre des composants d'agent de réticulation plurifonctionnels à base d'esters d'acide lactique et/ou d'acide glycolique qui, au moins partiellement, possèdent 2 à 4 - de préférence 2 et/ou 3 groupes oléfiniques dans la molécule.

19. Mode d'exécution selon les revendications 15 à 18, caractérisé en ce que comme composants d'agent de réticulation, on utilise des esters d'acide (méth)acrylique plurifonctionnels de produits réactionnels terminés par un hydroxyle, provenant de la réaction d'alcools plurifonctionnels inférieurs, en particulier l'éthylène glycol et/ou le glycérol, avec l'acide lactique et/ou l'acide glycolique.

20. Mode d'exécution selon les revendications 15 à 19, caractérisé en ce que les composants d'agent de réticulation ont été ou introduits par mélange dans le mélange support/substance de valeur et là ont été amenés à la réaction et/ou en ce qu'un mélange support/substance de valeur est revêtu avec une couche de matériau renfermant des composants durcisseurs qui ont réagi.

21. Mode d'exécution selon les revendications 15 à 20, caractérisé en ce que comme matériau support, des oligomères et/ou des polymères d'acides hydroxycarboxyliques inférieurs, compatibles avec le corps, en particulier à base d'acide lactique et/ou d'acide glycolique sont présents, qui possèdent de préférence des poids moléculaires moyens dans la zone environ de 200 à 5 000, en particulier environ de 300 à 2 000.
